# EUROPEAN PATENT SPECIFICATION

(11) **EP 1 576 094 B1**
(45) Date of publication and mention of the grant of the patent: **28.09.2011**
(21) Application number: 03724099.1
(22) Date of filing: 18.04.2003
(51) Int. Cl.: C12Q 1/68, C12N 1/20, C12N 15/00, C07H 21/02, C07H 21/04

(54) **METHODS OF CREATING MODIFIED PROMOTERS RESULTING IN VARYING LEVELS OF GENE EXPRESSION**
VERFAHREN ZUR ERZEUGUNG ZU VARIIERENDEN GENEXPRESSIONSNIVEAUS FÜHRENDER MODIFIZIERTER PROMOTOREN
METHODE DE CREATION DE PROMOTEURS MODIFIES PERMETTANT D'OBTENIR DIFFERENTS NIVEAUX D'EXPRESSION GENIQUE

(30) Priority: 22.04.2002 US 374735 P; 22.04.2002 US 374627 P
(43) Date of publication of application: 21.09.2005
(73) Proprietor: Danisco US Inc., Palo Alto, CA 94304 (US)
(72) Inventor: CERVIN, Marguerite, A., Redwood City, CA 94062 (US); VALLE, Fernando, Burlingame, CA 94010 (US)
(74) Representative: Kiddle, Simon John
(86) International application number: PCT/US2003/012044
(87) International publication number: WO 2003/089604

(56) References cited:
- WO-A-94/25609
- WO-A1-98/07846
- US-A- 5 532 142
- US-A- 6 140 087
- US-B1- 6 344 327
- ELLERMEIER C D ET AL: "Construction of targeted single copy lac fusions using lambda Red and FLP-mediated site-specific recombination in bacteria" GENE: AN INTERNATIONAL JOURNAL ON GENES AND GENOMES, ELSEVIER, AMSTERDAM, NL, vol. 290, no. 1-2, 1 April 2002 (2002-04-01), pages 153-161, XP004362098 ISSN: 0378-1119
- YU D ET AL: "AN EFFICIENT RECOMBINATION SYSTEM FOR CHROMOSOME ENGINEERING IN ESCHERICHIA COLI" PROCEEDINGS OF THE NATIONAL ACADEMY OF SCIENCES OF USA, NATIONAL ACADEMY OF SCIENCE, WASHINGTON, DC, US, vol. 97, no. 11, 23 May 2000 (2000-05-23), pages 5978-5983, XP002908442 ISSN: 0027-8424
- DATSENKO KIRILL A ET AL: "One-step inactivation of chromosomal genes in Escherichia coli K-12 using PCR products" PROCEEDINGS OF THE NATIONAL ACADEMY OF SCIENCES OF USA, NATIONAL ACADEMY OF SCIENCE, WASHINGTON, DC, US, vol. 97, no. 12, 6 June 2000 (2000-06-06), pages 6640-6645, XP002210218 ISSN: 0027-8424
- MEYNIAL-SALLES I ET AL: "New tool for metabolic pathway engineering in Escherichia coli: One-step method to modulate expression of chromosomal genes" APPLIED AND ENVIRONMENTAL MICROBIOLOGY, WASHINGTON,DC, US, vol. 71, no. 4, April 2005 (2005-04), pages 2140-2144, XP002367550 ISSN: 0099-2240
- MARTINEZ-MORALES F ET AL: "Chromosomal integration of heterologous DNA in Escherichia coli with precise removal of markers and replicons used during construction" JOURNAL OF BACTERIOLOGY, WASHINGTON, DC, US, vol. 181, no. 22, November 1999 (1999-11), pages 7143-7148, XP002156609 ISSN: 0021-9193

## Description

### FIELD OF INVENTION

The present invention relates to the genetic modification of bacterial cells. Particularly, the invention relates to a method of constructing a library of promoters that comprises precursor and modified precursor promoters, and use of the promoter library to replace the promoter of a chromosomal gene of interest in bacterial host cells, resulting in a population of bacterial host cells having a range of expression levels for the chromosomal gene of interest.

### BACKGROUND OF THE INVENTION

For many years microorganisms have been exploited in industrial applications for the production of valuable commercial products, such as industrial enzymes, hormones, and antibodies. Despite the fact that recombinant DNA technology has been used in an attempt to increase the productivity of these microorganisms, the use of metabolic genetic engineering to improve strain performance, particularly in industrial fermentations, has been disappointing.

A common strategy used to increase strain performance is to alter gene expression, and a number of means have been used to achieve this end. One approach includes the cloning of a heterologous or a homologous gene in a multi-copy plasmid in a selected host strain. Another approach concerns altering chromosomal gene expression. This has been accomplished by various methods some of which include: 1) site-specific mutations, deletions or insertions at a predetermined region of a chromosome; 2) reliance on transposons to insert DNA randomly into chromosomes; and 3) altering of native regulatory regions of a gene at its chromosomal location. The alteration of regulatory regions can be accomplished for example, by changing promoter strength or by using regulatable promoters which are influenced by inducer concentration. Reference is made to Jensen and Hammer, (1998) Biotechnology and Bioengineering 58:193 -195; Jensen and Hammer, (1998) Appl. Environ. Microbiol. 64:82 - 87; and Khlebnikov et al. (2001) Microbiol. 147:3241. Other techniques used to replace regulatory regions of chromosomal genes have been disclosed in Abdel-Hamid et al. (2001) Microbiol. 147:1483-1498 and Repoila and Gottesman (2001) J. Bacteriol. 183:4012 - 4023.

With respect to optimizing metabolic pathway engineering in a selected host, the above mentioned approaches have had limited success and each approach has certain disadvantages. Research has shown the expression level of a genetically modified gene on a plasmid is not necessarily correlated with the level of expression of the same modified gene located in the chromosome. (See Khlebnikov et al. (2001), Microbiol. 147:3241 and McCraken and Timms, (1999) J. Bacteriol. 181:6569).

Moreover, the effect of increasing expression of one gene in a metabolic pathway may have only a marginal effect on the flux through that metabolic pathway. This may be true even if the gene being manipulated codes for an enzyme in a rate-limiting step because control of a metabolic pathway may be distributed over a number of enzymes. Therefore, while a gene has been engineered to achieve a high level of expression, for example a 10 to 100 fold increase in expression, the overall performance of the engineered microorganism in a bioreactor may decrease. The decrease could be due to the balance of other factors involved in the metabolic pathway or the depletion of other substances necessary for optimum cell growth.

The above problem is addressed in part by Jensen and Hammer (WO 98/07846). The disclosure of WO 98/07846 describes the construction of a set of constitutive promoters that provide different levels of gene expression. Specifically, artificial promoter libraries are constructed comprising variants of a regulatory region that includes a -35 consensus box, a -10 consensus box and a spacer (linker) region that lies between these two consensus boxes. However, one of the drawbacks of the method described in WO 98/07846 is the extensive screening, which would be required of the promoter library. It is also disclosed in the reference that the modulation of promoter strength, by a few base pair changes in the consensus sequences or by changes in the length of the linker sequence, would result in a large impact in promoter strength, and therefore, it would not be feasible to achieve small steps in promoter strength modulation.

Therefore, a need still exists, in the area of metabolic pathway engineering, to develop a quick and efficient means of determining the optimum expression level of a gene in a metabolic pathway which in turn results in an optimization of strain performance for a desired product. The present invention satisfies this need by providing a method to characterize small changes in promoter strength of a modified precursor promoter and hence allowing for the selection of a cell providing an optimum level of gene expression.

### SUMMARY OF THE INVENTION

In one aspect the invention relates to a method of creating a library of bacterial cells having a range of expression levels of a chromosomal gene of interest which comprises obtaining a promoter library which includes at least two promoter cassettes, wherein the promoter cassette comprises in sequential order a 5' sequence homologous to an upstream flanking region of a target site; a first recombinase recognition site; a selectable marker; a second recombinase recognition site; precursor promoter comprising a -35 consensus region, a-10 consensus region and a linker sequence extending between the consensus regions, and a 3' sequence homologous to a downstream flanking region of the target site; transforming bacterial host cells with the promoter library, wherein the promoter cassettes are integrated into the bacterial host cells by homologous recombination to produce transformed host cells wherein the promoter cassette region of a gene of interest; culturing the transformed host cells under suitable growth conditions; and obtaining a library of transformed bacterial cells, wherein the transformed bacterial cells exhibit a range of expression levels of a chromosomal gene of interest. In one embodiment the method further comprises selecting transformed bacterial cells from the library. In a further embodiment, the selected transformed host cells will have a higher level of expression of the gene of interest than bacterial cells comprising the precursor promoter. In a second embodiment the selected bacterial cells have a lower level of expression of the gene of interest than the bacterial cells comprising the promoter of known sequence. Also described herein are transformed bacterial cells selected according to the method above. In further embodiments the promoter library comprises the Ptrc promoter and modified Ptrc promoters; the Ptac promoter and modified Ptrc promoters; and the P_{GI} promoter and modified P_{GI} promoters.

In a second aspect the invention relates to a vector comprising a promoter cassette comprising in sequential order a 5' sequence homologous to an upstream flanking region of a target site; a first recombinase recognition site; a selectable marker; a second recombinase recognition site; a modified precursor promoter comprising a -35 consensus region, a linker sequence and a -10 consensus region, wherein the modified promoter includes at least one modified nucleotide relative to a wild type promoter in a position corresponding to a -35 consensus region, a linker sequence or a -10 consensus region of a the wild type promoter; and a 3' sequence homologous to a downstream flanking region of the target site. The wild type promoter is selected from the sequences comprising base pairs -35 to +1 of the sequences in the group consisting of P*_{trc}*, P_{tacl}, P_{D/E20}, P_{H207}, P_{N25}, P_{G25}, P_{J5}, and wherein said at least two different promoter cassettes include: i) a first promoter cassette comprising a promoter of known sequence and a second promoter cassette comprising a promoter generated by modification of said known sequence; or ii) a first and second promoter cassette each comprising a promoter generated by modification of the same known sequence: P_{A1}, P_{A2}, P_{A3}, P_{L}, P_{lac}, P_{lacUV5}, P_{con}, P_{GI} and P_{bla}, the also disclosed hereins selection of the - 35 region of the precursor promoter from the group consisting of TTGACA, TTGCTA, TTGCTT, TTGATA, TTGACT, TTTACA and TTCAAA and the -10 region of the precursor promoter is selected from the group consisting of TAAGAT, TATAAT, TATACT, GATACT, AATAAT, TACGAT, TATGTT and GACAAT. In a further embodiment, a preferred promoter is Ptrc wherein at least one nucleotide is modified in either the -35 box, the - 10 box or the linker region. In a further embodiment, the promoter is P_{GI}, wherein at least one nucleotide is modified in either the -35 box, the - 10 box or the linker region.

In a further aspect, the invention relates to a promoter library comprising at least two promoter cassettes as described in the claims. In one embodiments, the invention relates to host cells transformed with a promoter cassette or a promoter library wherein the host cells are selected from the group consisting of *E. coli, Bacillus sp.* and *Pantoea sp.*

In yet another aspect, the invention relates to a method of modifying the regulatory function of a native promoter of a chromosomal gene of interest comprising, obtaining a promoter cassette according to the invention; transforming a host cell with the promoter cassette to allow homologous recombination between the promoter cassette and homologous flanking regions of a target site, wherein the promoter cassette replaces a native promoter region of a chromosomal gene of interest; and culturing the transformed host cells under suitable growth conditions. In one embodiment of this aspect, the selectable marker is excised from the transformed host cells, and in another embodiment the transformed host cells are further isolated.

In yet a further aspect, the invention concerns a method for altering the expression of a chromosomal gene of interest comprising obtaining a promoter cassette according to the invention; transforming a host cell with the promoter cassette; and allowing homologous recombination between the promoter cassette and homologous flanking regions of the target site, wherein the promoter cassette replaces a native promoter region of a chromosomal gene of interest and alters the expression of the chromosomal gene of interest as compared to the expression of the chromosomal gene of interest in a corresponding parent host cell.

### BRIEF DESCRIPTION OF THE DRAWINGS

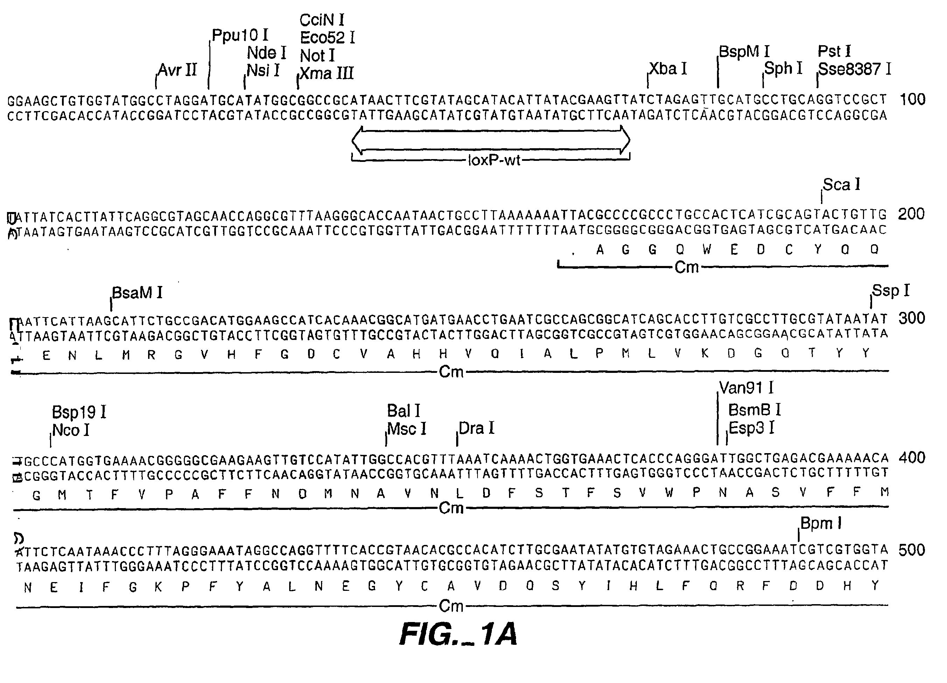

Figures 1A - 1K illustrate the nucleotide sequence of the pTrCm2 plasmid (SEQ ID NO. 1). The plasmid includes a first recombinase site and second recombinase site of IoxP and a chloramphenicol (cat or Cm) marker gene flanked on each side by IoxP. Further, the plasmid includes the *trc* promoter (P_{trc}) region comprising the -35 consensus box, the -10 consensus box and the linker sequence and a bla coding region for the beta-lactamase enzyme that confers ampicillin resistance. Additionally the amino acid sequence of the coding regions is illustrated (SEQ ID NO. 2).

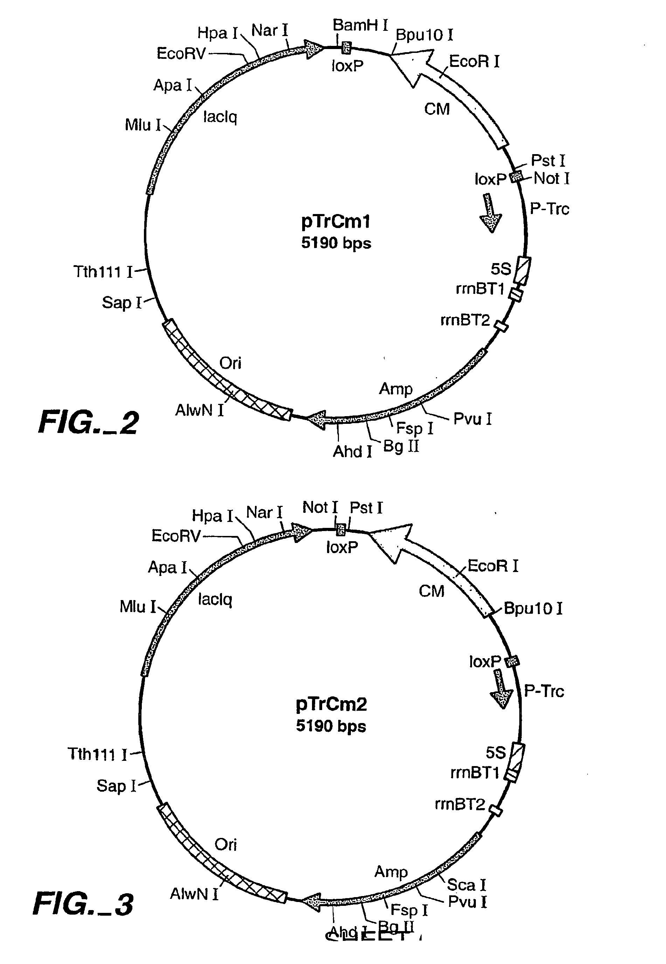

Figure 2 depicts a map of the TrCm2 plasmid.
Figure 3 depicts a map of the TrCm1 plasmid.

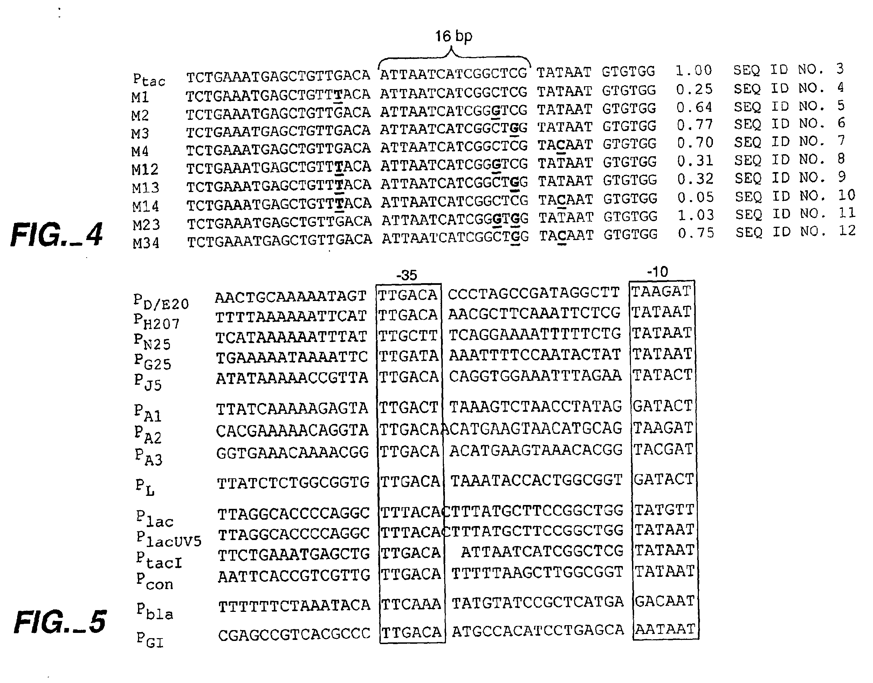

Figure 4 illustrates the nucleotide sequence of the promoter region and the relative promoter strength of the P_{tac} promoter and variants thereof having 1 or 2 base pair (bp) changes as disclosed in Sommer et al., (2000) Microbiology 146:2643-2653. P_{tac}, a chimeric bacterial promoter is represented by SEQ ID NO.3; variant M1 is represented by SEQ ID NO. 4; variant M2 is represented SEQ ID NO. 5; variant M3 is represented by SEQ ID NO. 6; variant M4 is represented by SEQ ID NO. 7; variant M12 is represented by SEQ ID NO. 8; variant M13 is represented by SEQ ID NO. 9; variant M14 is represented by SEQ ID NO. 10; variant M23 is represented by SEQ ID NO. 11 and variant M34 is represented by SEQ ID NO. 12.
Figure 5 illustrates the sequences of various well-characterized promoters and includes approximately 45 base pairs (bp) upstream of the transcriptional start site (+1), including the -35 consensus box, the linker sequence, and the -10 consensus box. The promoters are aligned with respect to the first T of the -35 consensus region and the last T of the -10 consensus region. The conserved regions are indicated by the boxes. P_{D/E20} is represented by SEQ ID NO. 13; P_{H207} is represented by SEQ ID NO. 14; P_{N25} is represented by SEQ ID NO. 15; P_{G25} is represented by SEQ ID NO. 16; P_{J5} is represented by SEQ ID NO. 17; P_{A1} is represented by SEQ ID NO. 18; P_{A2} is represented by SEQ ID NO. 19; P_{A3} is represented by SEQ ID NO. 20; P_{L} is represented by SEQ ID NO. 21; P_{lac} is represented by SEQ ID NO. 22; P_{lacUV5} is represented by SEQ ID NO. 23; P_{tacl} is represented by SEQ ID NO. 24; P_{con} is represented by SEQ ID NO. 25; P_{bla} is represented by SEQ ID NO. 26; and P_{GI} is represented by SEQ ID NO. 34.

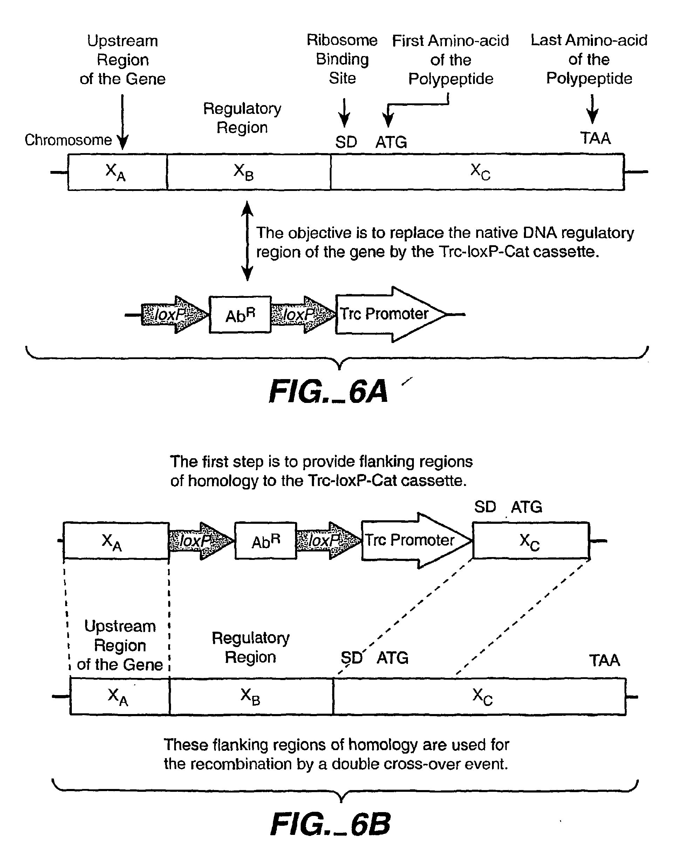

Figures 6A and 6B include a schematic representation according to the invention of a method used to replace the regulatory regions of a chromosomal gene of interest.
   A. A promoter cassette is constructed including a IoxP recombinase site, an Ab^{R} antibiotic marker, a second IoxP recombinase site and a P_{trc} promoter (which may be a modified P_{trc} wherein segments A, B and C are from the same gene. However, the A segment could be from a different gene or region non-relevant to the regulatory segment C.
   B. An upstream flanking region of homology (X_{A}) and a downstream flanking region of homology (X_{C}) to a chromosomal gene of interest is incorporated into the cassette. The flanking regions of homology are used for recombination by a double crossover event.
   C. The promoter cassette replaces the native regulatory region of the chromosomal gene of interest (Xc). The selective marker is excised from the chromosome and the final chromosomal structure includes a precursor or modified precursor promoter.

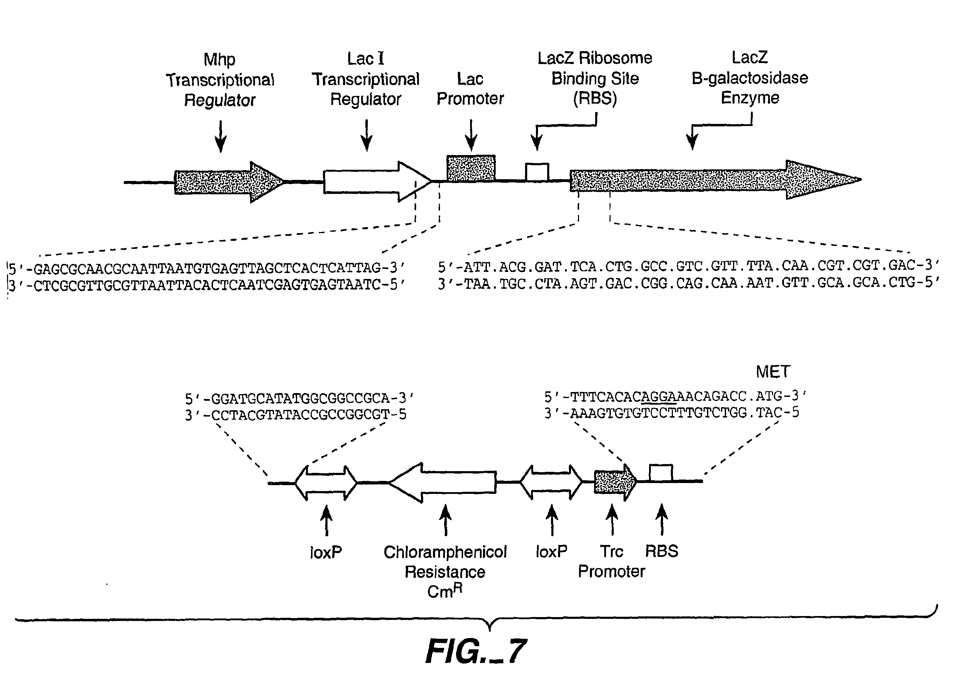

Figure 7 is a schematic representation illustrating the replacement of the Wild-type precursor lacZ promoter with a promoter cassette including an upstream nucleic acid fragment homologous to a 5' end of lacZ, a loxP recombinase site, a chloroamphenicol resistance gene, a second IoxP site, a trc promoter and a nucleic acid fragment homologous to a downstream region of the lacZ gene. Nucleic acid sequences represent double stranded DNA regions relevant to designing PCR primers; lacZ1 (SEQ ID NO. 41) and lacZ2 (SEQ ID NO. 42) (see example 4).

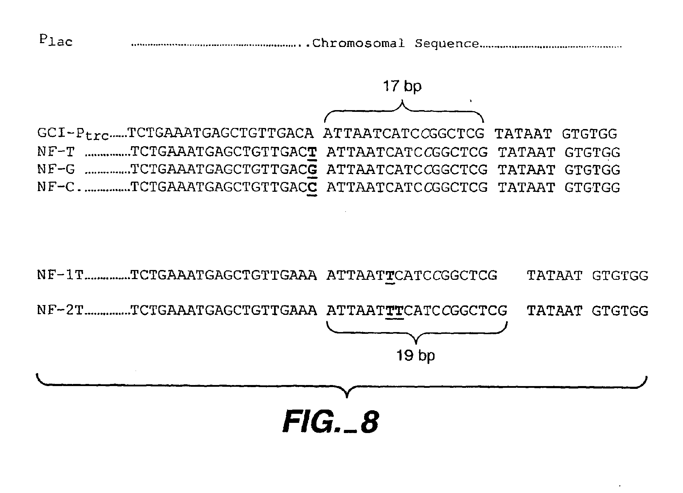

Figure 8 illustrates the nucleotide sequence of the precursor promoter Ptrc (SEQ ID NO. 27) and 7 modified precursor promoters wherein the precursor promoter is Ptrc. The-35 box of Ptrc is represented by TTGACA and the -10 box is represented by TATAAT. Modified precursor promoters NF-T (SEQ ID NO. 28), NF-G (SEQ ID NO. 29), and NF-C (SEQ ID NO. 30) include nucleotide base changes in the -35 box. The -35 box of NF-T is TTGACT, the -35 box of NF-G is TTGACG, and the -35 box of NF-C is TTGACC. Modified precursor promoters NF-1T (SEQ ID NO. 32) and NF-2T (SEQ ID NO. 33) include nucleotide base additions of "T" and "TT" respectively between ATTAAT and CATCCGGCT.... of the 17 bp sequence of Ptrc. Promoter strength is determined by beta-galactosidase activity in the presence and absence of the inducer isopropyl-beta-D-thiogalactopyranoside (IPTG) measured relative to the promoter strength of the control, chromosomal promoter Plac using a standard beta-galactosidase assay (Miller J.H. (1972) EXPERIMENTS IN MOLECULAR GENETICS. Cold Spring Harbor Laboratory Press pp 352 - 355).

### DETAILED DESCRIPTION OF THE INVENTION

One aspect of the present invention relates to the discovery that by modifying one or two nucleotides of a precursor promoter corresponding to nucleotides in the -35 consensus box, the -10 consensus box, or the linker region, promoter strength in terms of chromosomal gene expression could be changed to a level which allows quick identification of a range of gene expression. Furthermore, by constructing suitable promoter cassettes, the inventors were able to quickly test promoter efficiency at the chromosomal level.

### A. Definitions

In this application, unless otherwise stated, illustration of the techniques used may be found in any of several well-known references such as Sambrook, J., et al., MOLECULAR CLONING: A LABORATORY MANUAL, Cold Spring Harbor Laboratory Press (1989); Goeddel, D., ed., GENE EXPRESSION TECHNOLOGY, METHODS IN ENZYMOLOGY, 185, Academic Press, San Diego, Calif. (1991); Deutshcer, M. P., ed., GUIDE TO PROTEIN PURIFICATION, METHODS IN ENZYMOLOGY, Academic Press, San Diego, Calif. (1989); and Innis, et. al., PCR PROTOCOLS: A GUIDE TO METHODS AND APPLICATIONS, Academic Press, San Diego, Calif. (1990).

Unless defined otherwise, all technical and scientific terms used herein have the same meaning as commonly understood by one or ordinary skill in the art to which this invention pertains. Both Singleton et al., DICTIONARY OF MICROBIOLOGY AND MOLECULAR BIOLOGY, 2D ED., John Wiley and Sons, New York (1994) and Hale and Marham, THE HARPER COLLINS DICTIONARY OF BIOLOGY, Harper Perennial New York (1991) provide one of skill in the art with general dictionaries of many of the terms used in this invention. One is also directed to Sambrook et al., MOLECULAR CLONING: A LABORATORY MANUAL, Cold Spring Harbor Laboratory Press (1989) for definitions and terms of the art.

For the purposes of the present invention, the following terms are used to describe the invention herein.

A "promoter" or "promoter region" is defined herein as a nucleotide sequence, which is recognized and bound by a DNA dependent RNA polymerase during the initiation of transcription. In the context of the present invention a promoter includes two consensus regions generally hexamers. The first consensus region is centered about 10 base pairs (bp) upstream from the start site of transcription initiation and is referred to as the -10 sequence, -10 box or *Pribnow* box. The second consensus region is centered about 35 bp upstream of the start site and is referred to as the -35 sequence or -35 box. It is these two regions of homology in which the E. coli RNA polymerase is believed to recognize and functionally bind most tightly.

A linker sequence extends between each consensus sequence and is comprised of about 14 to 20 base pairs. With the exception of nucleotides -15, -16, -17 and -18, linker regions in general do not appear to have significant regions of homology larger than two bp from one promoter to another.

A promoter may be a regulatable promoter such as the trc promoter which is induced by IPTG or a constitutive promoter. Other sequences which are considered part of the regulatory region of a gene include the ribosomal binding site and transcription start site. The transcriptional start site means the first nucleotide to be transcribed and is designated +1. Nucleotides downstream of the start site are numbered +2, +3, +4 etc., and nucleotides in the opposite direction (upstream) are numbered -1, -2, -3 etc. The "ribosome binding site (RBS)" is a short nucleotide sequence usually comprising about 4-16 base pairs and is involved in the interaction of the mRNA with the ribosome for translation of an encoded protein.

A "precursor promoter" as used herein includes wild-type promoters and known variant promoters. A "wild-type" promoter is a naturally occurring promoter in either the plasmid or chromosome of a host organism (a non-mutant promoter), and the term is used interchangeability herein with "native" promoter. A variant promoter is a known mutant or modified wild type promoter including known hybrid promoters. A precursor promoter as used herein may be a wild-type promoter or a variant promoter.

A "modified precursor promoter" is a precursor promoter that has been modified by altering a nucleotide in at least one position corresponding to the -35 box, the -10 box or the linker region. A "promoter cassette" or "promoter construct" as used herein includes a precursor promoter or a modified precursor promoter. The term "promoter library" refers to a population of promoter cassettes wherein the population has at least two members. IA promoter library can be used, for example to generate a library of transformed host cells wherein members of the library (bacterial clones) have varying levels of promoter activity relative to the promoter activity of the precursor promoter. The varying levels of promoter strength result in a library of clones with different levels of expression for the same coding region of a gene of interest. Transformed host cells or bacterial clones may then be selected for optimal expression.

Modification or alteration may include addition (insertion), deletion or change (substitution) in at least one nucleotide base of a nucleic acid segment and particularly of a precursor promoter sequence. A "deletion" is defined as a change in one or more nucleotides wherein said nucleotides are absent. An "insertion" is the addition of one or more nucleotides as compared to the precursor promoter. A "substitution" results from the replacement of one or more nucleotides with a different nucleotide.

For the purpose of this invention a "tac promoter (Ptac)" also referred to as tacl in the literature is a precursor promoter comprising the nucleic acid sequence set forth in SEQ ID NO. 3 and SEQ ID NO. 24, wherein the -35 box is TTGACA, the linker is represented by 16 base pairs the -10 box is TATAAT (Brosius et al., J. Biol. Chem. 260:3539 (1985) and Deuschle et al., EMBO J 5:2987 - 2994 (1986)).

As used herein a "trc promoter (Ptrc)" ) is a precursor promoter comprising the nucleic acid sequence set forth in SEQ ID NO. 27. The nucleotide sequence of the -10 box and the -35 box is the same as Ptrc, but the linker section includes 17 bp. Ptrc differs from Ptac by the addition of a C nucleotide between nucleotides -18 and -19 of Ptac. Ptrc and Ptac are essentially identical in strength. (Russell and Bennett, Gene 20:231 (1982); Amann et al., (1983) Gene 25:167-178) and Mulligan et al., J. Biol Chem. 260:3529 (1985)).

A "gene" is defined herein as a sequence of nucleotides that code for a functional polypeptide or RNA molecule (regulatory RNA's, tRNA's, rRNA's). Genes may include both coding regions (exons), non-coding regions (introns) and regulatory regions such as promoters and enhancers.

The term "nucleic acid" includes RNA, DNA, and cDNA molecules. The term is used interchangeably with polynucleotide. An oligonucleotide is a short chain nucleic acid molecule. A primer is an oligonucleotide, whether occurring naturally as in a purified restriction digest or produced synthetically, which is capable of acting as a point of initiation of synthesis when placed under conditions in which synthesis of a primer extension product which is complementary to a nucleic acid strand is induced, (i.e. in the presence of nucleotides and an inducing agent such as DNA polymerase and at a suitable temperature and pH). The primer is preferably single stranded for maximum efficiency in amplification. The primer must be sufficiently long to prime synthesis of extension products in the presence of the inducing agent. In one embodiment primers are degenerate primers wherein one nucleotide base is modified relative to a sequence of a precursor promoter.

As used herein the term "polypeptide" refers to a compound made up of amino acid residues linked by peptide bonds. The terms "protein" and "polypeptide" are used interchangeably herein. It will be understood by one of skill in the art that as a result of the degeneracy of the genetic code, a multitude of nucleotides encoding a given protein may be produced.

A "DNA construct" refers to a sequence that is used to introduce a polynucleotide into a host cell. The definition of a DNA construct encompasses, for example a promoter cassette including a precursor promoter and/or a modified precursor promoter. In one embodiment, a DNA construct is used to integrate a polynucleotide into a chromosomal target site by homologous recombination. A DNA construct may include either homologous and/or heterologous sequences of a host cell gene. In one embodiment a DNA construct may be inserted into a vector.

The term "target site" is intended to mean a predetermined genomic location within a bacterial chromosome where the integration of a DNA construct is to occur.

The term "introduced" used in the context of inserting a nucleic acid into a cell means transfection, transformation, protoplast fusion, transduction or the like and includes reference to the incorporation of a nucleic acid into a prokaryotic cell where the nucleic acid may be incorporated into the genome of the cell, converted into an autonomous replicon or transiently expressed.

A "flanking region" or "flanking sequence" means any region or sequence that is either upstream or downstream of the sequence or region being discussed, e.g. for genes A, B, and C, gene B is flanked by A and C gene sequences. Homologous flanking regions are homologous (essentially identical) to a nucleic acid sequence in the host cell chromosome.

A "vector" refers to a nucleic acid construct designed for transfer between different host cells. A vector may include a DNA construct, plasmid, cloning vector, expression vector and bacteriophage.

A "recombinase recognition site" is a novel recombination site which facilitates directional insertion of nucleotide sequences into corresponding recombination sites at a target site in a chromosome.

As used herein a "selectable marker' or "selective gene" refers to a gene capable of expression in a host cell which allows for ease of selection of those host cells containing an introduced DNA construct with the selective marker. Typically a selective marker is a gene that confers antimicrobial resistance or a metabolic advantage on the host cell to allow cells containing an exogenous introduced DNA to be distinguished from cells that have not received the exogenous nucleic acid.

Chromosomal integration is a process wherein a DNA construct such as a promoter cassette is introduced into a host chromosome. The homologous flanking regions of the promoter cassette will align with homologous regions at the target site of the host chromosome.

"Homologous recombination" means the exchange of nucleic acid fragments between two DNA molecules or pair chromosomes (during crossing over) at the site of identical nucleotide sequences. In the present invention chromosome integration is preferably by homologous recombination.

A "metabolic pathway" is a series of chemical reactions that either break down a large molecule into smaller molecules (catabolism) or synthesize more complex molecules from smaller molecules (anabolism). Most of these chemical reactions are catalyzed by a number of enzymes. In many metabolic pathways there are rate-limiting enzymatic steps which serve to regulate the pathway. For example, in the glycolytic pathway wherein glucose is converted to pyruvate and ATP, phosphofructokinase is considered a key enzyme in regulation; and in the pentose phosphate pathway wherein NADPH and ribose-5-phosphate are generated, glucose -6-phosphate dehydrogenase and fructose 1,6-diphosphatase are considered key enzymes.

The term "homology" refers to sequence similarity or identity, with identity being preferred. Homology is determined using standard techniques known in the art. (Pearson et a., (1988) PNAS USA 85:2444 and Needleman et al., (1970) Adv. Appl. Math. 2:482).

As used herein the term "expression" refers to the process by which a polypeptide is produced based on the nucleic acid sequence of a gene. The process includes both transcription and translation. A "range of expression levels" means the plurality of expression levels of a gene of interest obtained from a library of bacterial clones transformed with a library of promoter cassettes.

As used herein, "optimal expression" refers to the cumulative conditions that provide an optimal level of gene expression for a particular coding region. Under certain conditions, optimal expression may mean a low level of gene expression.

"Operably linked" means that the nucleic acid sequences are functionally related. Generally operably linked means that the nucleic acid sequences being linked are contiguous. Linking is accomplished by ligation at convenient restriction sites. If such sites do not naturally exist, synthetic oligonucleotide adaptors or linkers are used in accordance with conventional practice.

"Isolated" as used herein refers to a nucleic acid or polypeptide that is removed from at least one component with which it is naturally associated.

"Host cell" means a cell that has the capacity to act as a host and expression vehicle for a promoter cassette or DNA construct. The host cell may be a recombinant host cell. A "corresponding parent host cell" means a bacterial cell that has not been transformed with a promoter cassette comprising a modified precursor promoter and which retains a precursor promoter. In general when a corresponding parent host cell and a transformed host cell comprising a modified precursor promoter are compared with respect to the level of gene expression, both cells will be grown under essentially the same growth conditions unless indicated otherwise.

As used herein the term polymerase chain reaction (PCR) refers to the methods of U.S. Patent Nos. 4,683,195; 4,683,202 and 4,965,188 which include methods for increasing the concentration of a segment of a polynucleotide in a mixture of DNA without cloning or purification. This process for amplifying a target sequence or DNA fragment consists of introducing two oligonucleotide primers to the DNA mixture containing the target sequence, followed by a sequence of thermal cycling in the presence of DNA polymerase. The two primers are complementary to their respective strands of the target sequence.

The term" heterologous nucleic acid" or "heterologous polypeptide" as used herein refers to a nucleic acid or polypeptide sequence that does not naturally occur in a host cell. With respect to a heterologous nucleic acid, the sequence has a portion which is not native to the cell in which it is expressed.

A "homologous nucleic acid" or "homologous polypeptide" as used herein refers to a nucleic acid or polypeptide that naturally occurs in a host cell.

When numeric ranges are used herein they are inclusive of the numbers defining the range.

As used in the specification the singular "a", "an" and "the" include the plural references unless the context clearly dictates otherwise. For example, the term "a cassette" may include a plurality of cassettes.

### B. Embodiments

Precursor promoters useful for creating a promoter cassette include the sequences of the precursor promoters listed in Table 1 below. Figure 5 illustrates the sequences of these precursor promoters including the -35 region, the -10 region and the linker region. All promoters in the table are characterized with respect to the β-lactamase promoter Pbla and promoter strengths are given in "Pbla-units". (Deuschle, et al., EMBO J.,5:2987-2994 (1986)).

**Table 1**

| PROMOTER | Source | Relative Activity | SEQ ID NO. |
|---|---|---|---|
| β-lactamase (bla) | *E.coli* vector | 1 | 26 |
| Pconsensus (con) | Synthetic DNA | 4 | 25 |
| PTac I (Trc) | Hybrid of 2 promoters | 17 | 24 |
| PLacUV5 | Mutant of Lac | 3.3 | 23 |
| PLac | *E.coli lacZ* gene | 5.7 | 22 |
| PL | Phage λ | 37 | 21 |
| PA1 | Phage T7 | 22 | 18 |
| PA2 | Phage T7 | 20 | 19 |
| PA3 | Phage T7 | 76 | 20 |
| PJ5 | Phage T5 | 9 | 17 |
| PG25 | Phage T5 | 19 | 16 |
| PN25 | Phage T5 | 30 | 15 |
| PD/E20 | Phage T5 | 56 | 13 |
| PH207 | Phage T5 | 55 | 14 |

In general precursor promoter sequences useful in the invention include sequences of between 200 to 20 bp, preferably of between 150 to 25 bp, more preferably of between 30 to 100 bp and most preferably between 50 to 30 bp upstream from the transcriptional start site (+1).

In one embodiment a preferred precursor promoter is a Trc promoter (Ptrc) wherein the -35 box is TTGACA, the linker is represented by 17 base pairs, and the -10 box is TATAAT (SEQ ID NO. 27) (Amann et al., (1983) Gene 25:167-178).

In another embodiment a preferred precursor promoter is a tac promoter (Ptac) (SEQ ID NOs. 3 and 24). The nucleotide sequence of the -10 box and the -35 box is the same in Ptac and Ptrc, but the linker region differs by 1 bp.

In another preferred embodiment, a precursor promoter is a P_{GI}. This promoter is also known in the literature as a xylose isomerase promoter and the regulatory sequence encompassing the promoter is disclosed in Amore et al., (1989) Appl. Microbiol. Biotechnol. 30:351 - 357. The sequence of the short segment of the promoter (+50 to -7 of the -10 box) is illustrated in SEQ ID NO. 34.
5' CGAGCCGTCACGCCCTTGACA ATGCCACATCCTGAGCA AATAAT, 3'
wherein the -35 box is represented by TTGACA and the -10 box represented by AATAAT.

A precursor promoter may be determined by various exemplary methods. While not wanting to be limited, in one embodiment, sequencing of a particular genome may be performed and putative promoter sequences identified using computerized searching algorithms. For example, by using Neural Network for Promoter Prediction software, NNPP. NNPP is a time-delay neural network consisting mostly of two feature layers, one for recognizing TATA-boxes (-10 boxes) and one for recognizing so called "initiators", which are regions spanning the transcription start site. Both feature layers are combined into one output unit. These putative sequences may then be cloned into a cassette suitable for preliminary characterization in *E*. *coli* and/or direct characterization in *E. coli*

Promoter sequences can also be identified by homology analysis. For example, a homology study of a family of genomes may be performed and analyzed for the presence of putative consensus promoters using BLAST. These putative promoter sequences may then be cloned into a cassette suitable for preliminary characterization in *E*. *coli*. Some preferred precursor promoters are listed in Figures 4 and 5.

A modified precursor promoter will comprise at least one modification to a nucleotide in a precursor promoter of known sequence. In one embodiments, the modification will be to a nucleotide base positioned in the -35 consensus region. This modification may include a modification to one or more nucleotide basel a position equivalent to the -30,-31, -32, -33, -34, and/or -35 position of a precursor promoter of known sequence. Preferably the modification will be of one nucleotide or two nucleotides and preferably the modification will be a substitution. When two positions are to be modified four positions will be conserved, and when one position is modified five positions will be conserved. In a further embodiment the modified precursor promoter will include a change at a position corresponding to -30 and/or a change to a position corresponding to -35.

Also disclosed herein is a modified precursor promoter is obtained from a precursor promoter having a -35 region represented by the following sequences, TTGACA, TTGCTA, TTGCTT, TTGATA, TTGACT, TTTACA and TTCAAA. Particularly preferred -35 consensus regions to be modified from a precursor promoter are TTTACA and TTGACA. As a nonlimiting example when TTGACA is the -35 box of the precursor promoter, the nucleotide at position -30 which is A may be substituted with a C, T, or G; the nucleotide at position -31 which is C may be substituted with a G, A or T; the nucleotide at position -32 which is A may be substituted with a T, C, or G; the nucleotide at position -33 which is G may be substituted with a C, T, or A; the nucleotide at position -34 which is T may be substituted with a C, G, or A; and/or the nucleotide at position -35 which is T may be substituted with a C, G or A. In one particular embodiment, the modified precursor promoter will include a modification of one to four nucleotides in the consensus region represented by TTGACA. In one embodiment four positions will be conserved and two positions will be modified. In another embodiment five positions will be conserved and one position will be modified. In a further embodiment the modified precursor promoter will include a change at a position corresponding to -30 or to a position corresponding to -35. When TTGACA is the precursor promoter it may be modified as follows, TTGACT, TTGACG, TTGACC or CTGACA.

In a further embodiment, the modification of the precursor promoter will be in the - 10 region. This modification may include a modification to one or more nucleotides at a position equivalent to the -7, -8, -9, -10 -11 or -12 position of a precursor promoter. Preferably, the modification will be in one or two nucleotide positions. In a preferred embodiment, the modification will be a substitution at one or two nucleotide positions. Also disclosed herein are preferred precursor promoters including the following sequences in the -10 region: TAAGAT, TATAAT, TATACT, GATACT, TACGAT, AATAAT, TATGTT and GACAAT. Particularly preferred -10 regions include the sequences AATAAT, TATAAT, TATGTT and TAAGAT. In one embodiment the precursor promoter is Ptrc (SEQ ID NO. 27) and the modified precursor promoter will include at least one modification of a nucleotide in the -10 region represented by TAAGAT. For example the nucleotide at position -7 which is T may be substituted with G, C or A; the nucleotide at position -8 which is A, may be substituted with T, C or G; the nucleotide at position -9 which is G, may be substituted with C, T or A; the nucleotide at position -10 which is A may be substituted with T, C or G; the nucleotide at poison -11 which is A may be substituted with T, C or G and the nucleotide at position -12 which is T may be substituted with T, C or G. In one embodiment four positions will be conserved and two positions will be modified. In another embodiment five positions will be conserved and one position will be modified.

In some embodiments of the invention both the -35 region and the -10 region of the precursor promoter may have modifications. In one embodiment, the modification will include one nucleotide position in the - 35 box, wherein the other nucleotides remain conserved, and will include a modification at one nucleotide position in the -10 box wherein the other nucleotides remain conserved. In another embodiment, the modification will comprise a modification to a -35 region represented by TTGACA and a -10 region represented by TATAAT, and in another embodiment, the modification will comprise a modification to a -35 region represented by TTGACA and a -10 region represented by AATAAT. The total number of modifications in a -35 box and -10 box of a precursor promoter may include one position in each consensus box, wherein the other positions are conserved. The total number of modifications in a -35 box and -10 box of a precursor promoter may also include one position in one consensus box and two positions in the other consensus box, wherein the other positions are conserved. Also the total number of modifications in a -35 box and -10 box of a precursor promoter may include two positions in each consensus box, wherein the other positions are conserved.

In a further embodiment a modified precursor promoter includes a modification to the linker sequence of the precursor promoter of known sequence. Linker sequences are in general 14 to 20 nucleotides, and more typically 16 to 18 nucleotides. A modification may include the addition of 1, 2, 3, 4, or 5 base pairs to a linker sequence or the substitution of 1, 2, 3, 4, 5 or more base pairs. Preferably the modification is the addition of one or two base pairs in the linker region wherein the addition may be any one of the nucleotides of A, T, C or G. Preferably the addition comprises one or two base pairs to the linker sequence of Ptrc. Further the addition to Ptrc preferably occurs between base pair -23 and -24. Further embodiments include the addition of T (see SEQ ID NO. 32) and TT between base pairs - 23 and -24 (see SEQ ID NO. 33). In a further embodiment the modification may include the substitution of one, two or three nucleotide bases in any position of the precursor linker sequence. Preferably the modification is the substitution of one nucleotide base in any position of the precursor linker.

In further embodiments a modified precursor promoter includes a modification to the -35 box, the linker region and the -10 box. The modification will include at least three nucleotide positions and no more than eight nucleotide positions wherein each region includes one modification. Preferably the modification will include three nucleotide positions wherein each region includes one modification.

The modified precursor promoter sequence may be generated by means well known in the art including but not limited to mutagenesis techniques including chemical mutagenesis, polymerase chain reaction and site-directed mutagenesis to one or more nucleotides. (see Miller, J. H. A. A SHORT COURSE IN BACTERIAL GENETICS, Cold Spring Harbor Laboratory Press 1992). In one embodiment degenerate oligonucleotides are synthesized for the host cell for which a promoter library is to be constructed. In a preferred embodiment, alteration to the precursor promoter is accomplished by site-directed mutagenesis using the QuickChange commercial kit (Stratagene, La Jolla, CA).

In one embodiment specific promoters, which have been constructed according to the invention, include those modified P_{trc} promoters: NF-T (SEQ ID NO. 28); NF-G (SEQ ID NO. 29); NF-C (SEQ ID NO. 30); NF-1T (SEQ ID NO. 32) and NF-2T (SEQ ID NO. 33). Another promoter is the promoter designated MC-C3 having the sequence
TCTGAAATGAGCTGCTGACA ATTAATCATCCGGCTCG TATAAT GTGTGG (SEQ ID
NO. 31) wherein the -35 box is CTGACA, the linker region is ATTAATCATCCGGCTCG and the -10 box is TATAAT.

A promoter cassette will include a precursor promoter and/or a modified precursor promoter as disclosed above. Further a promoter cassette may include a 5' sequence homologous to an upstream flanking region of a target site wherein the target site is preferably a chromosomal gene of interest. A 5' sequence homologous to an upstream flanking region of a target site may include from 5 to 500 nucleotides, preferably from 10 to 200 nucleotides, also from 10 to 100 nucleotides and additionally 10 to 50 nucleotides, which are homologous to the nucleotides upstream of the target site.

The gene of interest may be any chromosomal gene. In one embodiment the gene of interest encodes a therapeutically significant protein such as growth factors, cytokines, hormones, ligands, receptors and antibodies. In another embodiment the gene of interest encodes a commercially important enzyme such as amylases, proteases, glucoamylases, dehydrogenases, esterase, cellulases, galactosidases, oxidases, reductases, kinases, xylanases, laccases, phenol oxidases, glucose oxidases, catalases, lipases and phytases. In further embodiments the gene of interest encodes transporter proteins, such as glucose and/or galactose permease (transporters). In other embodiments, the gene of interest may encode enzymes in a metabolic pathway, such as glucose dehydrogenase, pyruvate dehydrogenase and pyruvate oxidase. In particular embodiments the gene of interest will encode industrially important proteins such as lipases, esterases, hydrogenases and proteases. The chromosomal gene of interest or encoding region thereof may be heterologous or homologous to the host cell but will be operably linked to a native promoter or precursor promoter of known sequence which will be replaced by a library of promoters or by a modified precursor promoter

A promoter cassette may also include two recombinase recognition sites and a selectable marker flanked by each recombinase recognition site. Examples of recombinase recognition sites are well known in the art. Recombinases generally fall into two distinct families that each use a different mechanism of catalysis. These are the tyrosine recombinases and the serine recombinases. Either type of recombinase system may be used in the present invention. The tyrosine recombinase family is also known as the lambda-integrase family and includes 100 or more identified members. (Nunes-Duby, et al, Nucleic Acids Research 26:391 - 406 (1998)). There are more than 72 serine recombinases described in the literature and these include Tn3, Hin, SOPIVCA, φC31 and λ. Particularly preferred recombinases which could be used in the invention include Cre and Flp (Nunes-Duby, D, et al, Nucleic Acids Research 26:391- 406 (1998) and Huang et al., Nucleic Acids Research 19:443 (1991)); XerC-XerD (cer, parB dif and psi) (Blake et al., (1997) Mol. Microbiol. 23:387 - 398); P22 xis-int (AttP22 and ataA) (Cho et al. (1999) J. Bact. 181:4245 - 4249); SPOIVCA (SpoIVCB, SpoIIIC, AttPskin and AttBskin) (Straiger et al., (1989) Sci. 243:507-512); Resolvase (res) (Yang and Steitz (1995) Cell 82:193 - 207) and λInt (Att, attL and attR) (Hallet and Sherrat (1997) FEMS Microbiol. Lett. 21: 157 - 178).

Particularly preferred recombinases are Cre and Flp. In a most preferred embodiment, the first and second recombinase recognition sites include the bacteriophage P1 Cre/*loxP* recombination system, which comprises a Cre enzyme and two asymmetric 34 bp *loxP* recombination sites (See Sternberg and Hamilton (1981) J. Mol. Biol. 150:467 - 486; Van Duyne (2001) Ann. Rev. Biophys. Biomol. Struct. 30:87 - 104 and Palmeros, B, et al Gene 247:255 (2000)). A *loxP* site comprises two 13 bp sequences, inverted and imperfectly repeated, which surround an 8 bp core asymmetric sequence, where crossing-over occurs. The Cre-dependent intramolecular recombination between two parallel *loxP* sites results is excision of any intervening DNA sequence as a circular molecule, producing two recombination products, each containing one *loxP* site. Preferably, the promoter cassette includes a selective marker flanked by two loxP sites. In a particularly preferred embodiment the recombinase sequence will include variants of the loxP site because if two LoxP sites are in the right orientation in the chromosome, they can promote the loop-out or inversion of big regions of the chromosome. Reference is made to Sauer E., Curr. Opin. Biotech. (1994), 5: 521-527; Palmeros et al. Gene 247 (2000) 255-264. and Hoess et al.,(1986) NAR, 14:2287-2230).

In a preferred embodiment a selectable marker is located between the two recombinase sites. While various selectable markers may be used a preferred selective marker is an antibiotic resistance gene. These are well known in the art. For example, the gene may be an erythromycin resistance gene (Em^{r}), an ampicillin resistance gene (Ap^{r}), a chloramphenicol resistance gene (Cm^{r}), gentamicin resistance gene (Gm^{r}) or a kanamycin resistance gene (Km^{r}). Preferably once the promoter cassette including the selectable marker is introduced into a host cell, the marker is removed for example by following the teaching of Palmeros et al. (2000) Gene 247 (2000) 255-264.

A promoter cassette may also include a 3' sequence homologous to a downstream flanking region of the target site. The 3' sequence may include from 5 to 500 nucleotides, also 10 to 200 nucleotides, also 10 to 100 nucleotides and further 10 to 50 nucleotides which are homologous to the nucleotides downstream of a target site.

The DNA constructs including the promoter cassettes may include various restriction sites facilitating the ligation of various fragments of the DNA construct. Restriction sites may include Xbal, EcoRI, BgIII, BamHI, Taql and the like. For example restriction sites may be used for ligation with the -35 sequence fragment and the translation start codon and gene sequences positioned downstream therefrom.

The promoter cassettes and modified precursor promoter nucleic acids may also include other sequence such as ribosome binding sites (RBS), mRNA stabilizing sequences, enhancers, silencing sequences, transcriptional terminators, transcriptional attenuators, operators and mRNA destabilizing sequences.

In some embodiments the precursor promoter and the chromosomal gene of interest are heterologous and in other embodiment the precursor promoter and the chromosomal gene of interest are homologous.

Promoter cassettes may be constructed using standard well-known recombinant engineering techniques such as PCR and as described in various references such as Sambrook *supra.*, Palmeros et al. (2000) Gene 247:255 - 264; Datsenko and Wanner (2000) Proc. Natl. Acad. Sci. USA 10:640 - 6645). In general a promoter is cloned into a vector and a selected marker is linked to the promoter by cloning the marker upstream of the promoter in such a manner that the cloning step does not influence the function of the promoter. The resulting marker-promoter region is used as a promoter cassette. The cassette may be isolated by PCR or with restriction enzymes. To allow for homologous recombination, the cassette may be linked to regions of homology with the host cell chromosome using PCR by incorporating regions of homology in PCR primers. Or by ligating proper DNA restriction fragments (Datsenko and Wanner (2000) Proc. Natl. Acad. Sci. USA 10:6640-6645).

In other embodiments certain portions of the modified promoter may be deleted or excised from the promoter cassette, and the modified precursor promoter re-tested. In the event that expression is observed after this modification, and determination of whether expression has increased or decreased following modification, a positive or negative regulatory element of the promoter may be identified. In addition, specific regions of the precursor promoter may be isolated and tested in isolation. In this manner, specific elements may be identified that regulate gene expression in the host cell.

The invention further includes a library of promoters. A library will comprise at least two promoter cassettes. However, a promoter library may comprise 10³ or more members. In preferred embodiments, the promoter library will comprise at least 2, at least 3, at least 4, at least 8, at least 16, at least 64 members. Preferably the library will comprise promoter cassettes wherein the modified precursor promoters are obtained from the same precursor promoter of known sequence. In one embodiment a library of promoters may include promoter cassettes comprising modified Ptrc and precursor Ptrc. In another embodiment a library of promoters swill include promoter cassettes comprising modified Ptac and precursor Ptac, and in another embodiment a library of promoters will include promoter cassettes comprising a modified P_{GI} and a precursor P_{GI}. Preferred precursor promoters include those mentioned above for the promoter cassette. In another embodiment the library will comprise promoter cassettes herein the modified precursor promoter is obtained from different precursor promoters of known sequence.

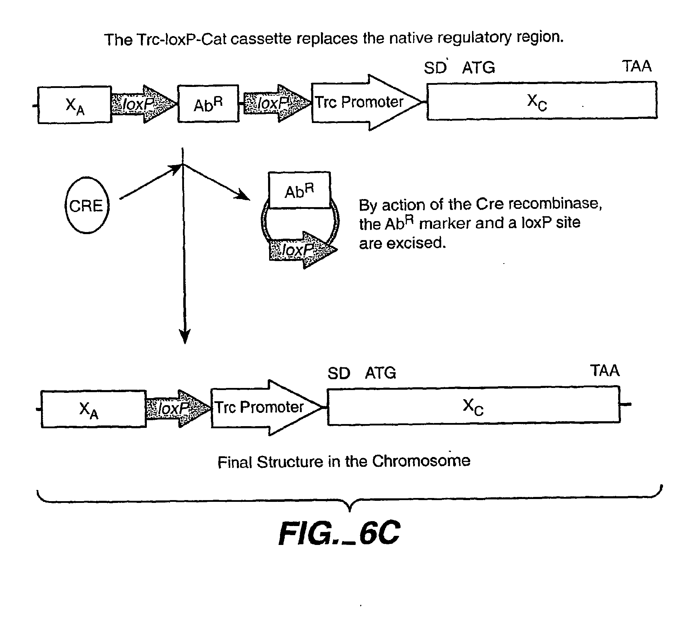

Promoter cassettes according to the invention may be used individually and introduced into a host cell or may be used in a promoter library. Figures 6 and 7 schematically illustrate the construction of a promoter cassette, the introduction of the promoter cassette into a host, the replacement of the host regulatory region with the promoter cassette and excision of the selective marker according to the invention.

In one embodiment a host cell is a bacterial cell. A bacterial host cell may be a gram-positive cell. Preferably the host cell is a *Bacillus* species. *Bacillus* species include but are not limited to *B. subtilis*, *B. licheniformis*, *B. lentus*, *B. brevis, B. stearothermophilus*, *B. alkalophilus, B. amyloliquefaciens, B. clausii, B. halodurans, B. megaterum, B. coagulans*, *B. circulans*, and *B. thuringiensis.*

In another embodiment a host cell is a gram-negative bacterial cell, such as an *Escherichia* species or *Pantoea* species. *E. coli* are the most preferred host cells. The genus of *Pantoea* includes all members known to those of skill in the art, including but not limited to *P. citrea, P. terrea, P. agglomerans, P. dispersa, P. punctata, P. ananas* and *P. stewartii.* It is recognized that the genus Pantoea continues to undergo taxonomical reorganization. Thus, it is intended that the genus include species that have been reclassified including but not limited to such microorganisms as *Erwinia herbicola.*

Preferably promoter cassettes are introduced into host cells by transformation. General methods for transformation are well known and reference is made to CURRENT PROTOCOLS IN MOLECULAR BIOLOGY, vol. 1 eds. Ausubel et al., (1987) Chapter 7, John Wiley & Sons. Transformation techniques include electroporation, use of calcium chloride and rubidium chloride. (Maniatis et al., (1982) MOLECULAR CLONING: A LABORATORY MANUAL, chapter 8. Cold Spring Harbor Laboratory, *supra*) and Potter, H (1988) Anal. Biochem 174:361 - 373).

Methods suitable for the growth and maintenance of host cells are also well known and reference is made to the MANUAL OF METHODS OF GENERAL BACTERIOLOGY, Eds. P. Gerhardt et al., America Society for Microbiology, Washington, DC (1994) and T.D. Brock in BIOTECHNOLOGY: A TEXTBOOK OF INDUSTRIAL MICROBIOLOGY 2Ed. (1989) Sinauer Associates, Sunderland, MA. Typically cells are grown at 35°C in appropriate media. Preferred growth media are common commercially prepared media such as Luria Bertani broth (LB), Sabouraud Dextrose (SD) or other known growth media.

Transformed host cells comprising a precursor promoter or modified precursor promoter are selected based upon the phenotype response to a selectable marker, which was provide with the promoter cassette. In some embodiments of the invention the selective marker is excised from the transformed host cells. Reference is made to Figure 6B and Palmeros et al. *supra.* In specific embodiments the IoxP site is left upstream of the promoter (for example in Figure 6B Ptrc. If the IoxP site is left in the host it could become a problem if the marker excision process is repeatable. If two IoxP sites are in the right orientation in the host chromosome, they may promote loop-out or inversion of large regions of the host chromosome (See Sauer, (1994) Curr. Opin. Biotech 5:521 - 527). To solve this problem, there is also disclosed the construction of IoxP-cat constructs that contain variants of the IoxP site that do not recombine efficiently with the wild-type IoxP site. It is known that the IoxP wild-type and the IoxP511 sites do not recombine with each other in a Cre-dependent manner (Hoess et al. (1986) NAR, 14:2287 - 2230). Other non-competitive IoxP sites are known.

Transformed host cells having an optimum level of gene expression may be selected and further isolated. Optimization of gene expression in host cells is achieved by selecting transformed host cells having between about 1 to 250%, between about 5 to 200%, between about 10 to 150%, and between about 10 to 100% the strength or expression of the precursor promoter. That is about 1%, 5%, 10%, 15%, 20%, 25%, 40%, 50%, 60%, 80% 100%, 150%, 200% and 250% or more of the strength of the precursor promoter.

Promoter strength can be quantified using in vitro methods that measure the kinetics of binding of the RNA polymerase to a particular piece of DNA, and also allows the measurement of transcription initiation. Reference is made to Hawley D.K et al., Chapter 3: in: PROMOTERS: STRUCTURE AND FUNCTION IN R.L. Rodriguez and M.J. Chamberlin eds. Praeger Scientific. New York. In vivo methods may also be used to quantify promoter strength. For example a promoter may be fused to a reporter gene and the efficiency of RNA synthesis measured. (Deuschle et al., (1986) EMBO J. 5: 2987-2994). The strength of *E. coli* promoters using different reporter genes was measured by Deuschle et al. and the data is presented in Table 1 above. Moreover, promoter strength may be defined in a number of ways. One common method is a relative one, wherein the mRNA or protein expressed by one gene per unit of time is compared to a control where the same gene is expressed by a different promoter. For example, the relative level of expression of the IacZ gene when it is transcribed by its native promoter (P_{Iac}) or from the P_{IacV5} promoter.

In an embodiment of the invention, promoter cassettes may be used in a method of modifying the regulatory function of a native promoter of a chromosomal gene of interest or by altering expression of a chromosomal gene of interest by transforming host cells with one or more promoter cassettes (a promoter library) and allowing homologous recombination between a promoter cassette and homologous flanking regions of a target site of the chromosomal gene of interest in the host cell, wherein the promoter cassette replaces a native promoter region of the chromosomal gene of interest.

Different promoter cassettes comprising modified precursor promoters derived from the same precursor promoter of known sequence will produce transformed host cells having a varying range of gene expression levels. The promoter strength and/or gene expression may be determined by various known methods. Promoter strength or gene expression may be compared between different transformed host cells and a parent having a precursor promoter a known sequence when cultured under essentially the same growth conditions. A transformed host having a desired level of expression may then be selected and isolated. The level of expression may be lower or higher than the expression of the same gene in a control parent having a native or precursor promoter of known sequence.

Additionally, selected transformed host cells may be chosen from a promoter library wherein the expression of the gene of interest from the modified precursor promoter is between about 1 to 250%, between about 5 to 200%, between about 10 to 150%, and between about 10 to 100% the strength of the original precursor promoter of known sequence.

Using a promoter library to create a population of bacterial cells having varying levels of expression of a gene of interest is particularly useful in a metabolic engineering pathway framework. Metabolic engineering is being used to optimize the metabolic pathways of strains to overproduce biomolecules. These biomolecules could be intermediates of cellular metabolism, polypeptides RNAs, carbohydrates, lipids and others.

Furthermore, the synthesis of complex molecules such as steroids, antibiotics, and other pharmaceuticals may require complicated and multiple catalytic pathways.

In an isolated system, each step in a particular metabolic pathway would need to be engineered. In contrast, the microorganism utilized in a whole cell system provides each of the required pathways. However, the use of certain promoters may incur problems in that a particular promoter may be too strong. As a result, the over expression of a particular gene may occur and be detrimental to a cell, for instance the gene may be expressed to the exclusion of other genes. The cell viability can thus be reduced and the production time may be limited.

The methods provided herein are utilized to provide a library of promoters to be introduced into bacterial host cells, which results in a population of transformed bacterial cells having a range of gene expression. The range of expression is useful because it allows the selection of specific bacterial clones having an optimum level of expression but still maintaining cell viability (e.g. the flux production of the desired end product relative the viability of the host cell in sustaining the desired level of production or sustaining the desired level of production). In certain embodiments the optimum level of expression of a gene will be high and in other embodiments the optimum level of gene expression will be low. A direct advantage of this method is that a bacterial clone may be selected based on the expression level obtained by the modified precursor promoter and then be ready for use in a fermentation process whereby cell viability is not negatively affected by expression of the gene of interest.

The following Examples are for illustrative purposes only and are not intended, nor should they be construed as limiting the invention in any manner.

### EXAMPLES

### EXAMPLE 1 - Construction of a Trc-IoxP-Cat promoter cassette.

An excisable selectable marker was introduced upstream of the Trc promoter by the following method. The commercial plasmid pTrc99a (Pharmacia) was digested with the restriction enzymes Hind III and Ncol according to supplier instructions (New England Biolabs). The digested DNA was purified and then submitted to a fill-in reaction with T4 DNA polymerase as described by Maniatis et al. *supra.* The resulting blunt-end linear DNA was re-circularized according standard protocols (Sambrook et al., *supra*) and the resulting ligation mixture transformed into *E.coli* TOP-10 competent cells (Invitrogen). The cells were plated on LB-agar plates containing 50 micrograms/ml of carbenicillin. After 16 hrs of incubation at 37 °C, a number of colonies appeared on the plate. Four of the colonies were chosen for further analysis.

Purified plasmid DNA was obtained from these colonies and subjected to restriction enzymes analysis. It was confirmed that the 4 colonies contained the same plasmid and that the DNA region between HindIII and Nco I was deleted. This plasmid was named pTrc1.

Plasmid pTrc1 contained only one recognition site for the restriction enzyme BspM1, located approximately 120 base pairs upstream of the -35 region of the Trc promoter. This location was selected to introduce the excisable selectable marker. pTrc1 was digested with the BspM1 enzyme according to the instructions of the supplier (New England Biolabs). The linear pTrc1 was gel-purified using a QIAquick gel extraction kit (QIAGEN), and submitted to a fill-in reaction with T4 DNA polymerase as described by Maniatis et al. *supra.* The resulting blunt-end linear DNA was ligated to a DNA construct including a chloramphenicol resistance gene (cat) flanked by IoxP sites. This construct was obtained from plasmid pLoxCat2 (Palmeros et al., Gene 247 (2000) 255-264) by digestion with Ssp1 and Bam H1. The Ssp1-Bam H1 DNA fragment was gel purified and blunt ended. Linear pTRC1 and the Ssp1-BamHI fragments were ligated. The ligation mixture was transformed into *E.coli* TOP-10 competent cells (Invitrogen) and plated on LB-agar plates containing 50 micrograms/ml of carbenicillin and 20 micrograms/ml of chloramphenicol. After 16 hrs. of incubation at 37 °C, several colonies appeared on the plate. Some of these colonies were transferred to a fresh LB-plate containing Carbenicillin and Chloramphenicol. After plasmid purification and restriction enzyme analysis, two clones containing the IoxP-cat cassette in both orientations were selected. These plasmids were named pTrCm1 and pTrCm2 (Figures 2 and 3).

An important consideration of the pLoxCat1 and 2 vectors described here, is that they still contain the lac operator that allows the binding of the Lacl repressor and provide certain degree of regulation for the Trc promoter (Amann et al, (1988) Gene, 69 301-315.)

### EXAMPLE 2. Construction of modified Trc promoters in the -35 box

There are numerous methods for DNA mutagenesis, the procedure described here is used to exemplify the process, but is not restricted to it. The QuickChange site-directed mutagenesis kit (Stratagene) was chosen to introduce a small number of modifications (mutations) in a defined region of DNA. This method is based on the use of polymerase chain reaction (PCR) to mutagenize a template (normally a plasmid) and the process requires two primers. After several PCR cycles many copies of the template are produced. Each copy was primed by the mutagenic primers. The PCR reaction is then treated with the restriction enzyme Dpn1 that only cuts the original template (non-mutagenized) in many sites. The PCR-products are insensitive to Dpn1. After the Dpn1 treatment, the resulting DNA is used to transform *E.coli* competent cells. The recovered transformants normally are highly enriched for the mutants.

To change the second A (position -31) of the TTGACA sequence of the -35 region, 2 mutagenic primers were designed and synthesized by a commercial supplier (Operon technologies Inc.).

Primer A:
5'-CTGAATGAGCTGTTGACNATTAATCATCCGGCTCG-3' (SEQ ID NO. 35) and
Primer B:
   5'-CGAGCCGGATGATTAATNCTCAACAGCTCATTTCAG 3' (SEQ ID NO. 36)
wherein "N" indicates T, G or C.

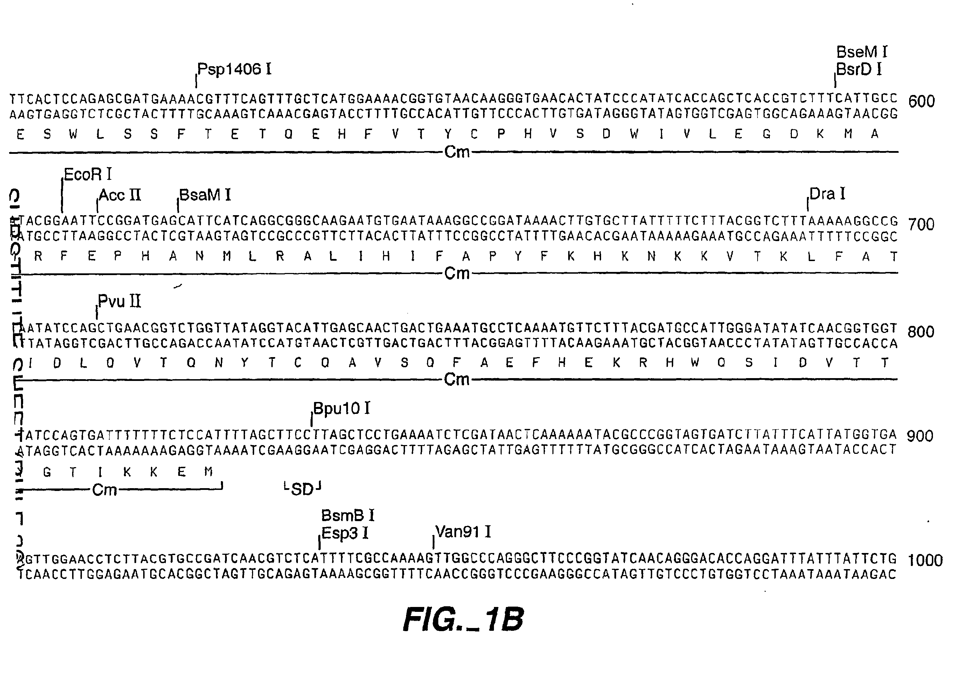

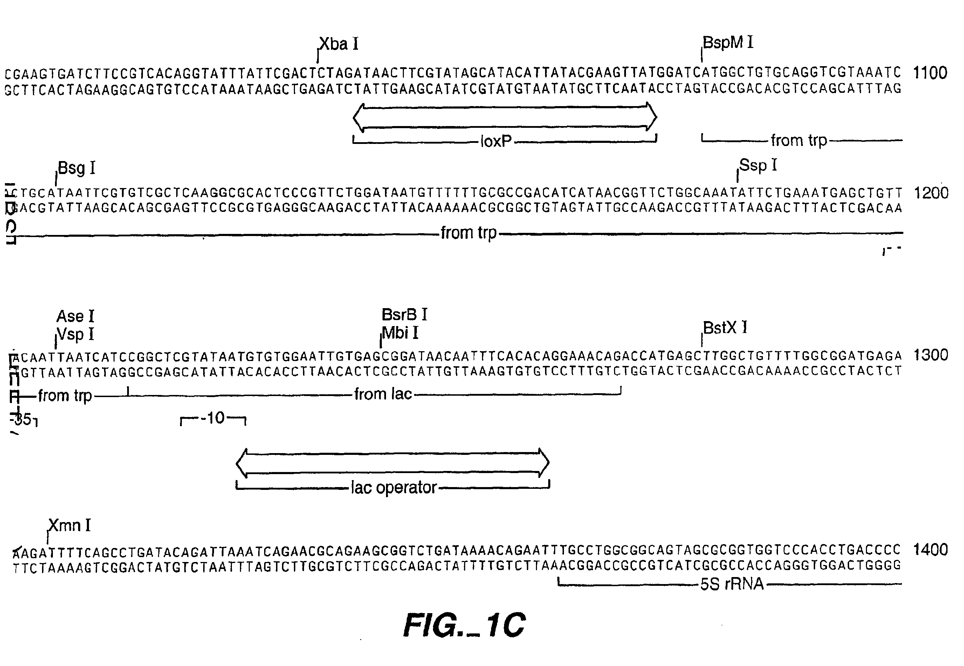

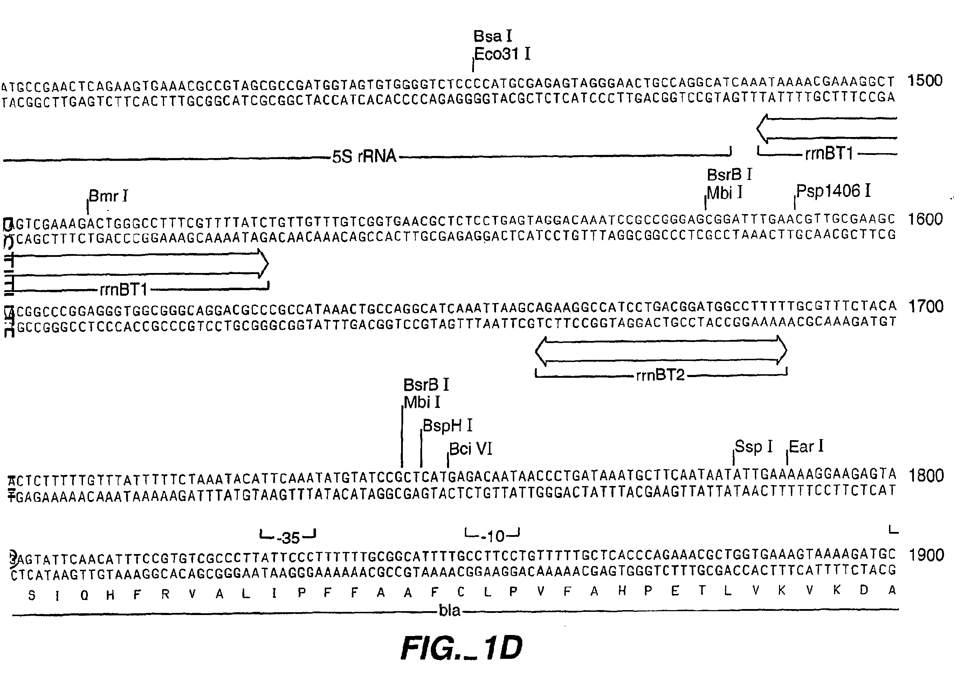

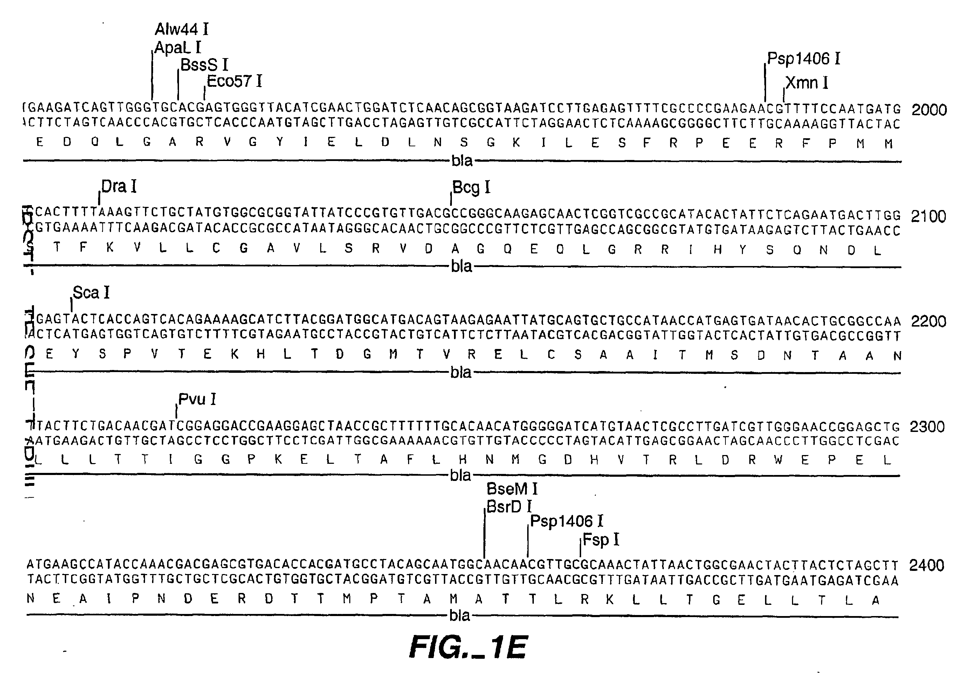

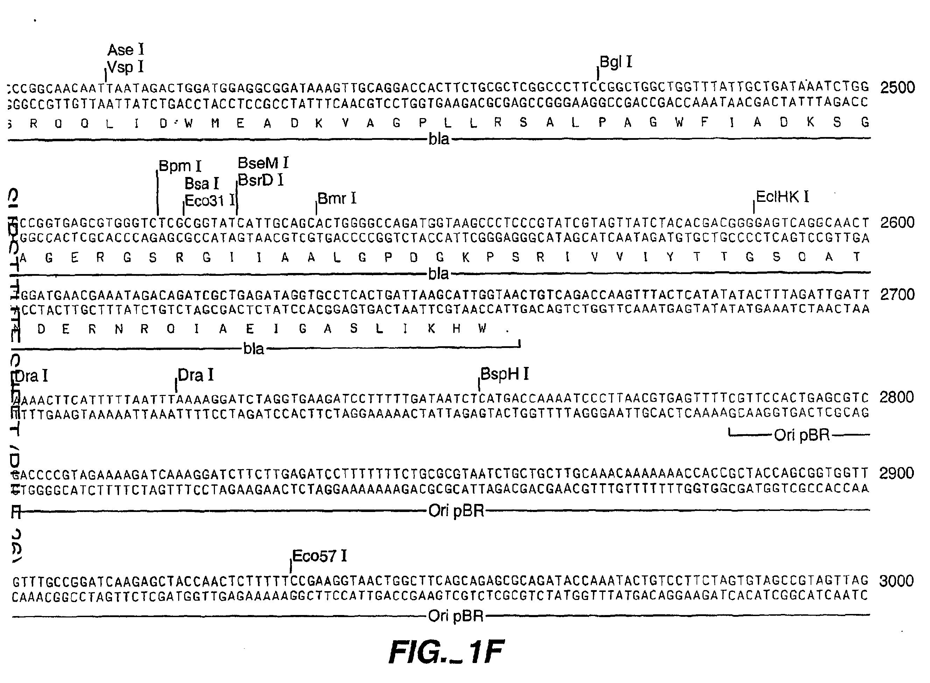

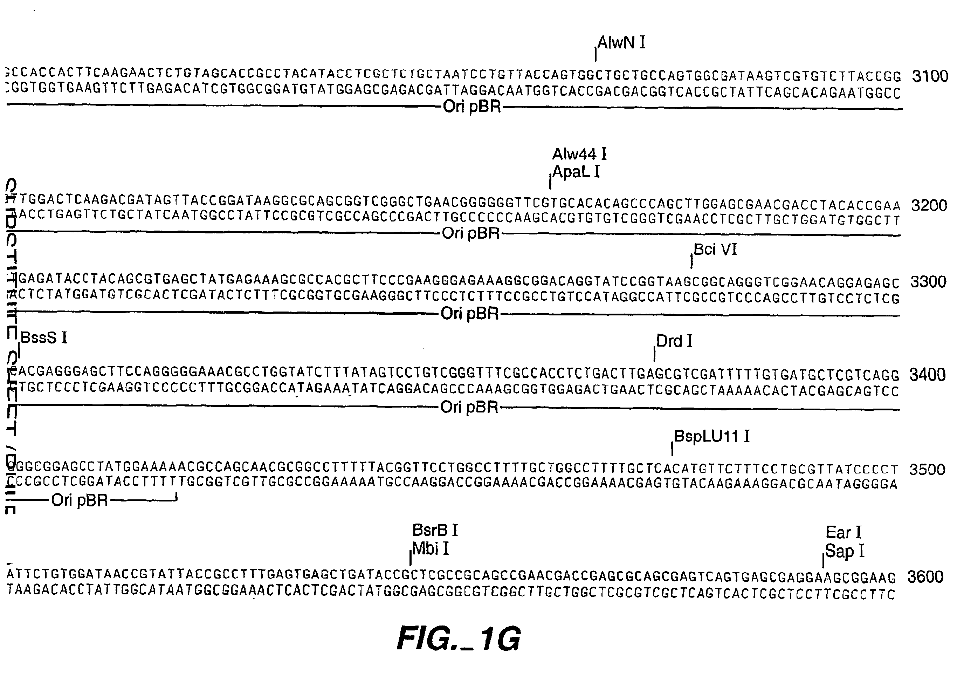

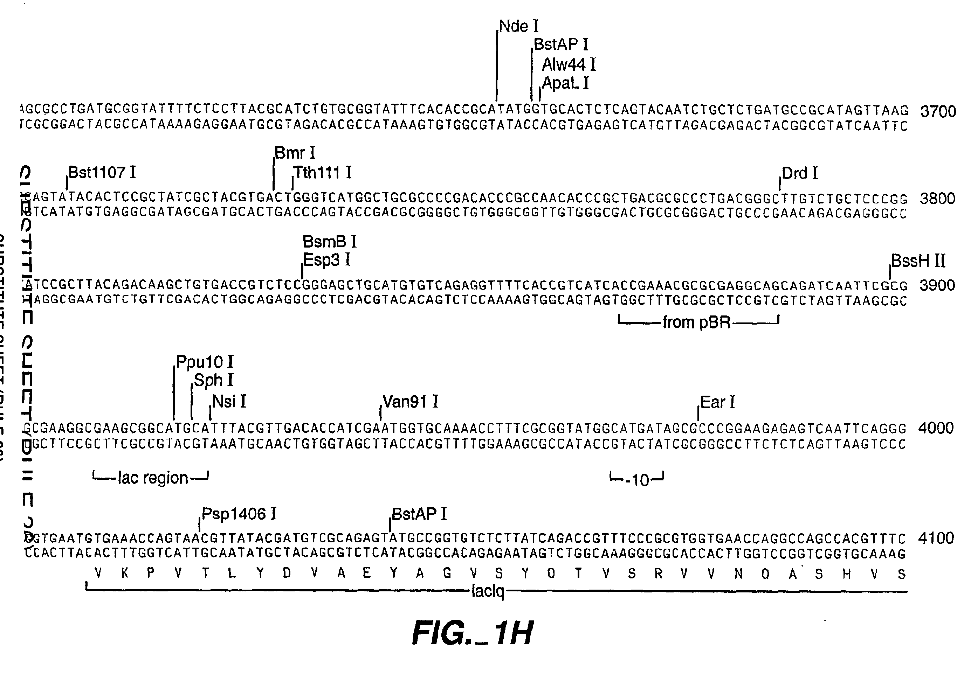

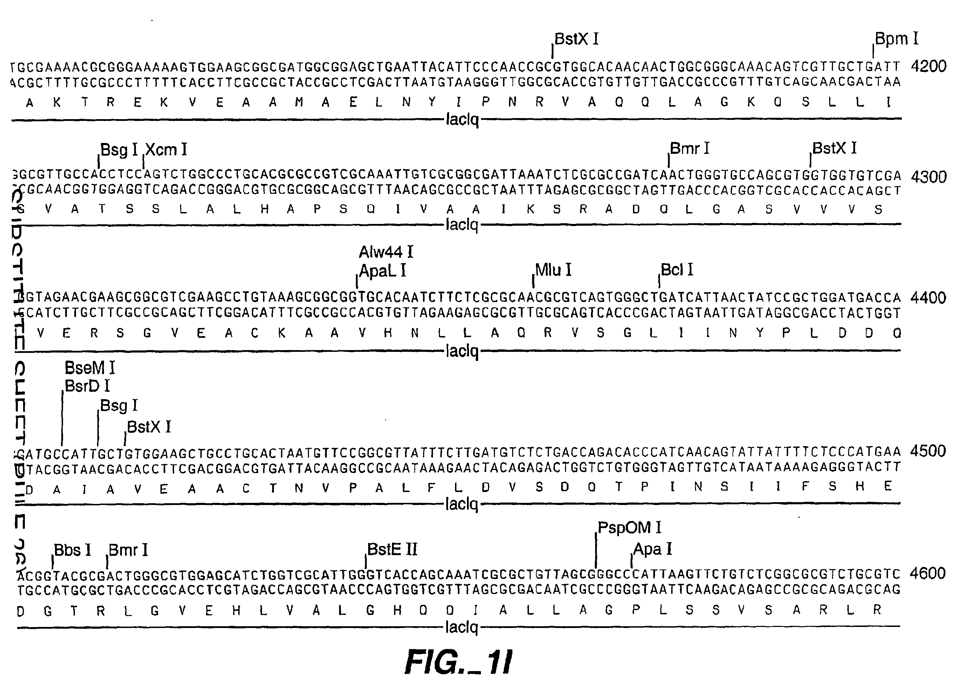

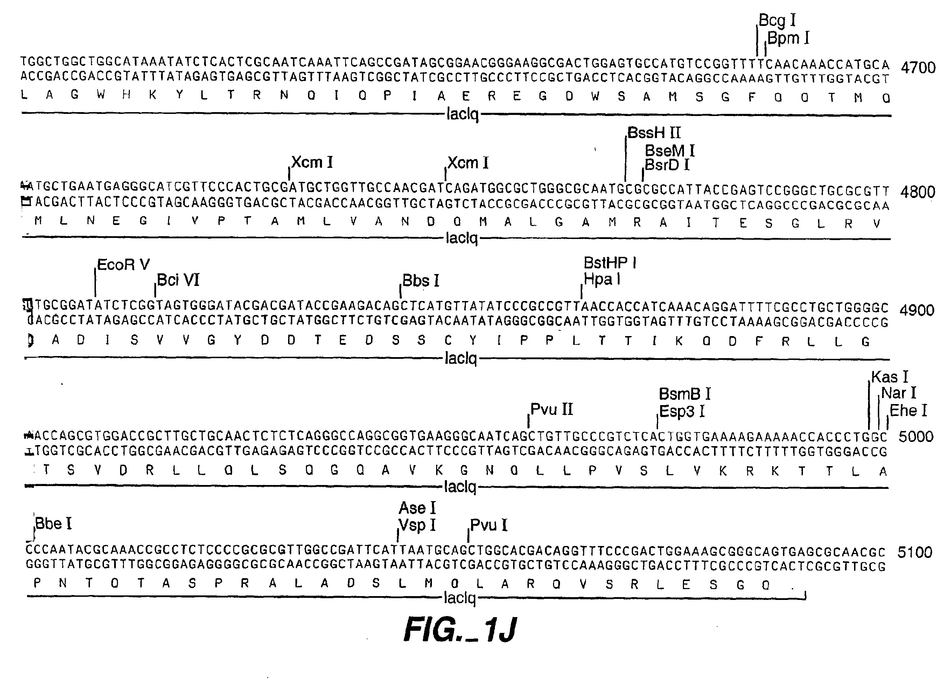

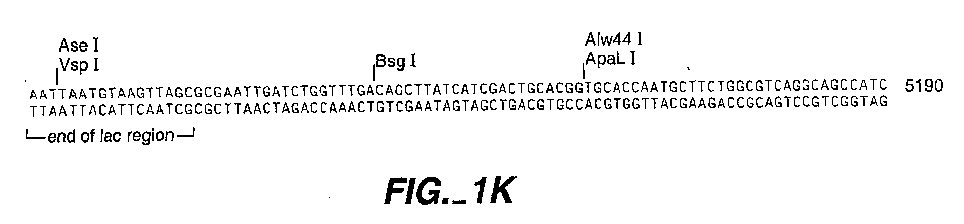

These two primers were used for the mutagenesis using the pTrCm2 plasmid (Figures 1 and 2) as a template, and the QuickChange kit, following the procedure recommended by the supplier. The QuickChange kit is provided with its own transformation protocol and competent cells (*E. coli* strain XL1 blue) that after transformation normally produce numerous transformants.

After transformation and plating on selective plates, plasmid DNA was purified from several clones and submitted for sequencing. In this manner modified precursor promoters were identified. i.e: promoters containing a -35 box with the sequence: Clone NF-T (SEQ ID NO. 28) TTGACT= plasmid pTrcCm31T
Clone NF-C (SEQ ID NO. 30) TTGACC = plasmid pTrCm31C
Clone NF-G (SEQ ID NO. 29) TTGACG= plasmid pTrCm31 G

Reference is made to Figure 8.

### EXAMPLE 3. - Construction of modified Trc promoters in the spacer region between the -10 and -35 boxes.

To demonstrate that a small number of modifications are enough to provide a range of levels of promoter strength in the linker region, the normal spacing of the Trc promoter was increased 1 bp at the time. i.e., 1 or 2 bases were added using the QuickChange protocol described in Example 2 using the following primers:
Pair for pTrCm 18 
5'-GACAATTAATTCATCCGGCTCG-3' (SEQ ID NO. 37)
5'-CGAGCCGGATGAATTAATTGTC-3 (SEQ ID NO. 38)
Pair for pTrCm19

5'-GACAATTAATTTCATCCGGCTCG-3' (SEQ ID NO. 39)
5'-CGAGCCGGATGAAATTAATTGTC (SEQ ID NO. 40)

The modified (mutant) plasmids were named as follow:
Clone NF-1T (SEQ ID NO 32) pTrCm18: spacer length =18
Clone NF-2T (SEQ ID NO. 33) pTrCm19: spacer length =19

EXAMPLE 4. - Use of pTrCm2 to replace chromosomal regulatory regions.

### Replacement of the IacZ promoter

The method disclosed in Datsenko and Wanner (Proc,. Natl. Acad. Sci. USA, 10: 6640-6645, (2000)) was utilized to replace the native regulatory regions of the lacZ gene with Ptrc and modified Ptrc promoters This method utilizes 30 - 50 nucleotides as regions of homology to promote homologous recombination between PCR products and the *E. coli* host cell chromosome. Plasmid TrCm2 and its derivatives were used as templates for the PCR reactions,
Primers 
lacZ1 (SEQ ID NO. 41) - 5' – AGCGCAACGCAATTAATGTGAGTTAGCTCAC
TCATTAGGGATGCATATGGCGGCCGCA -3'
 and
   lacZ2 (SEQ ID NO. 42) - 5' 
GTCACGACGTTGTAAAACGACGGCCAGTGAATCCGTAATCATGGTCTGTTTCCTGTGT
GAAA – 3'
 were designed to contain 20 nucleotides complementary to the pTrcCm2 and 39 nucleotides complementary to the lacZ gene regulatory region (Figure 7). Using these primers, a 1333bp DNA fragment was generated by PCR.

*E. coli* strain, MG1655 was transformed with plasmid pKD46 as recommended by Datsenko and Wanner (*supra*). The resulting strain MG1655/pKD46 was used to prepare competent cells according to the method described in Datsenko and Wanner, supra. Competent cells (100 µl) were transformed by electroporation with 20 to 100 ng of the 1333 bp PCR product described above. After recovering the cells for 1 hr in 1.0ml SOC media (sterile 10ml of 1M MgCl₂ and sterile 20 ml of 1 M glucose is added to autoclaved 20g tryptone, 5g yeast extract, 0.5g NaCl, and 2.5 ml 1M KCI), they were plated on 4 LA plates, containing 10µg/ml of chloramphenicol. Plates were incubated at 37C for at least 16 hrs. CM^{R} colonies were transferred to fresh LA plates containing 10 µg/ml chloramphenicol. To verify that the native regulatory region of the lacZ gene has been modified chromosomal DNA from the MG1655 and some of the Cm^{R} transformants were purified using the UltraClean microbial DNA isolation kit (MO BIO Labs, Solana Beach, CA). These DNAs were used as substrates for PCR reactions using primers.
LacT1 (SEQ ID NO. 43)
   5' GGCACGACAGGTTTCCCGAC - 3'
   and
lacT2 (SEQ ID NO. 44)
   5' GAGGGGACGACGACAGTATC 3'

These two primers hybridize with regions outside of where lacZ1 and lacZ2 hybridize and should generate PCR products of the following sizes: MG1655-425bp and MG1655:: Ptrc-Cm-Iacz-1585 bp (and 1586 bp and 1587 bp for the pTrCm18 and pTrCm19 cassettes.

The PCR products were separated in a 2% agarose gel. Based on the results of the gel colonies with proper modifications to the lacZ regulatory region were identified.

The proper integration of the cassettes can be further corroborated by sequencing the PCR products.

Furthermore, plasmids pTrcCm31T, pTrcCm31C and pTrcCm31G described in example 2, can be also used with the same purpose. It is expected that the cassettes of pTrCm1 and pTrCm2 will provide higher levels of expression than the cassettes from pTrcCm31T, pTrcCm31C or pTrcCm31G. It is also expected that the pTrCm18 and pTrCm19 promoters are weaker than the wt trc promoter.

### EXAMPLE 5. - Measurement of the expression of the lacZ gene.

The lacZ codes for the β-galactosidase enzyme in *E. coli* and this gene has been widely used as a reporter to quantify gene expression. The β-galactosidase activity is measured using the synthetic substrate ONPG (ortho-Nitrophenyl- β-D-galactoside) according to the procedure described by Miller, J.H., (A SHORT COURSE IN BACTERIAL GENETICS. Cold Spring Harbor Laboratory Press,1992), To quantify the level of expression of the lacZ gene in the strains described in example 3, the strains were grown overnight in Luria broth (LB) media. These overnights were used to inoculate 250 ml flask containing 50 ml of LB or LB+ 100uM IPTG. As a reference point, strain MG1655 was also inoculated. In the case of MG1655, lacZ will be expressed from its native promoter.

Flasks were incubated in a shaker at 37 °C until they reached early -exponential phase (-0.8 OD at 600 nm) and 1.5 samples were collected by centrifugation. The cells were resuspended in 1 ml of buffer Z and the β-galactosidase activity assayed as described by Miller J,H, (A SHORT COURSE IN BACTERIAL GENETICS. Cold Spring Harbor Laboratory Press, 1992). After correcting for the volumes utilized in the assay, the relative activity of β-galactosidase per unit of optical density (600 nm) was calculated. The results of these measurements are presented in Table 2.

**TABLE 2**

| Promoter controlling LacZ expression | SEQ ID NO. | LB Relative β-galactosidase activity | LB+100µM IPTG Relative β-galactosidase activity |
|---|---|---|---|
| wt lac | 22 | - | 1 |
| Trc | 27 | 1.46 | 4.78 |
| TrCm31T | 28 | 0.81 | 4.13 |
| TrCm31G | 29 | 1.47 | 5.01 |
| TrCm31C | 30 | 0.50 | 3.82 |
| TrCm18 | 32 | 0.82 | 2.23 |
| TrCm19 | 33 | 0.038 | 0.40 |

Table 2. Relative β-galactosidase activity measured in strains containing the lacZ gene under the control of different promoters.

### EXAMPLE 6. - Construction of a LoxP-Cat cassette containing a LoxP variant.

Sequences of the wild type and IoxP511 are shown below. The 2 IoxP sites differ in only one base pair.
LoxP wild type:
   5' ATAACTTCGTATAATGTATGC.TATACGAAGTTAT 3' (SEQ ID NO. 45) and LoxP511
5' ATAACTTCGTATA.ATGTAT**A**C.TATACGAAGTTAT 3' (SEQ ID NO. 46).

To construct the LoxP511-Cat cassette, two 2 primers containing the mutated base were used to amplify the IoxP-Cat cassette by PCR. This approach (and others) can be used to construct any variant of the LoxP sequence.

The sequences of the PCR primers are:
LoxF1: 5'-GCTGGATCCATAACTTCGTATAATGTATACTATACG-3' (SEQ ID NO. 47)
   and
LoxF2: 5'-GCATATGGCGGCCGCATAACTTCGTATAGTATACATT-3' (SEQ ID NO. 48).

PCR was performed and the PCR product was cloned in the vector pCR-Blunt II TOPO, following the instructions provided with the vector-kit (Invitrogen). E. coli cells were transformation and many colonies were obtained. After plasmid purification and restriction analysis, 3 colonies with the correct restriction pattern, were submitted for DNA sequencing and it was found that one plasmid presented the correct LoxP511 sequence. This plasmid was named pLoxCat27. In one embodiment this cassette would be used with a modified precursor promoter or precursor promoter when a promoter cassette is being introduced into a bacterial host strain that may already have a LoxP site.

## Claims

1. A method of creating a library of bacterial cells having a range of expression levels of a chromosomal gene of interest comprising,
a) obtaining a promoter library comprising at least two different promoter cassettes, wherein the promoter cassettes comprise in sequential order:
a 5' sequence homologous to an upstream flanking region of a target site;
a first recombinase recognition site;
a selectable marker;
a second recombinase recognition site;
a promoter comprising a -35 consensus region, a -10 consensus region and a linker sequence extending between the consensus regions, and wherein said at least two different promoter cassettes include: (i) a first promoter cassette comprising a promoter of known sequence and a second promoter cassette comprising a promoter generated by modification of said known sequence; or (ii) a first and second promoter cassette each comprising a promoter generated by modification of the same known sequence; and
a 3' sequence homologous to a downstream flanking region of the target site,
b) transforming bacterial host cells with the promoter library, wherein the promoter cassettes are integrated into the bacterial host cells by homologous recombination between the promoter cassettes and the flanking regions of the target site to produce transformed host cells, wherein the promoter cassette replaces a native promoter region of a chromosomal gene of interest;
c) culturing the transformed host cells under suitable growth conditions;
and
d) obtaining a library of transformed bacterial cells, wherein the transformed bacterial cells exhibit a range of expression levels of the chromosomal gene of interest.

2. The method according to claim 1 further comprising selecting transformed bacterial cells from the library.

3. The method according to claim 1, wherein the host cells are selected from the group consisting of *E. coli*, *Bacillus* sp. and *Pantoea* sp.

4. The method according to claim 2, wherein:
(a) the selected bacterial cells have a higher level of expression of the gene of interest than bacterial cells comprising the wild type promoter; or
(b) the selected bacterial cells have a lower level of expression of the gene of interest than the bacterial cells comprising the wild type promoter.

5. The method according to claim 1, wherein:
(a) the promoter library comprises the Ptrc promoter and modified Ptrc promoters; or
(b) the promoter library comprises the Ptac promoter and modified Ptrc promoters; or
(c) the promoter library comprises the P_{GI} promoter and modified P_{GI} promoters; or
(d) the promoter library comprises modified promoters having SEQ ID NO. 28, SEQ ID NO. 29 and SEQ ID NO. 30.

6. A vector comprising a promoter cassette, the promoter cassette comprising in sequential order
(a) a 5' sequence homologous to an upstream flanking region of a target site;
(b) a first recombinase recognition site;
(c) a selectable marker;
(d) a second recombinase recognition site;
(e) a modified promoter comprising at least one modified nucleotide relative to a wild type promoter in a position corresponding to a -35 consensus region, a -10 consensus region or a linker sequence extending between the consensus regions of the wild type promoter, wherein the wild type promoter is selected from the group consisting of P*_{trc}*, P_{tacl}, P_{D/E20}, P_{H207}, P_{N25}, P_{G25}, Pj₅, P_{A1}, P_{A2}, P_{A3}, P_{L}, P_{lac}, P_{lacUV5}, P_{con}, and P_{bla}; and
(f) a 3' sequence homologous to a downstream flanking region of the target site, wherein homologous recombination between the promoter cassette and the flanking regions of the target site results in the promoter construct replacing a native promoter region of a chromosomal gene of interest.

7. The vector of claim 6, wherein the linker sequence is modified.

8. The vector of claim 6, wherein said first and said second recombinase recognition sites are non-identical recombinase sites and selected from lox and mutant lox sites.

9. The vector of claim 6, wherein the modified promoter is selected from the group consisting of SEQ ID NO. 28 (NF-T), SEQ ID NO. 29 (NF-G), SEQ ID NO. 30 (NF-C), SEQ ID NO. 32 (NF-1T) and SEQ ID NO. 33 (NF-2T).

10. A promoter library comprising at least two different promoter cassettes, wherein the promoter cassettes comprise in sequential order:
a 5' sequence homologous to an upstream flanking region of a target site;
a first recombinase recognition site;
a selectable marker;
a second recombinase recognition site;
a promoter comprising a -35 consensus region, a -10 consensus region and a linker sequence extending between the consensus regions, and wherein said at least two different promoter cassettes include: (i) a first promoter cassette comprising a promoter of known sequence and a second promoter cassette comprising a promoter generated by modification of said known sequence; or (ii) a first and second promoter cassette each comprising a promoter generated by modification of the same known sequence; and
a 3' sequence homologous to a downstream flanking region of the target site.

11. A host cell transformed with a vector of any one of claims 6 to 9.

12. The host cell of claim 11, wherein the host cell is selected from the group consisting of *E. coli*, *Bacillus sp.* and *Pantoea sp.*

13. A method of modifying the regulatory function of a native promoter of a chromosomal gene of interest comprising;
a) obtaining a vector comprising a promoter cassette according to any one of claims 6 to 9;
b) transforming a host cell with the vector comprising a promoter cassette to allow homologous recombination between the promoter cassette and homologous flanking regions of a target site, wherein the promoter cassette replaces a native promoter region of a chromosomal gene of interest; and
c) culturing the transformed host cells under suitable growth conditions.

14. The method according to claim 13 further comprising;
(a) excising the selectable marker from the transformed host cell; and/or
(b) isolating the transformed host cells.

15. The method according to claim 1 further comprising;
(a) isolating transformed host cells having a desired expression level of a gene of interest and/or
(b) excising the selectable marker from the transformed host cell.

16. A method for altering the expression of a chromosomal gene of interest comprising;
a) obtaining a vector comprising a promoter cassette according to any one of claims 6 to 9;
b) transforming a host cell with the vector comprising a promoter cassette;
and
c) allowing homologous recombination between the promoter cassette and homologous flanking regions of the target site, wherein the promoter cassette replaces a native promoter region of a chromosomal gene of interest and alters the expression of the chromosomal gene of interest as compared to the expression of the chromosomal gene of interest in a corresponding parent host cell.

## Patentansprüche

1. Verfahren zur Erstellung einer Bibliothek von Bakterienzellen mit einem Bereich von Ausmaßen an Expression eines chromosomalen Gens von Interesse, umfassend:
a) das Erhalten einer Promotorenbibliothek, die zumindest zwei verschiedene Promotorkassetten umfasst, worin die Promotorkassetten Folgendes in aufeinander folgender Reihenfolge umfassen:
eine 5'-Sequenz, die zu einer stromauf flankierenden Region einer Zielstelle homolog ist;
eine erste Rekombinaseerkennungsstelle;
einen selektierbaren Marker;
eine zweite Rekombinaseerkennungsstelle;
einen Promotor, der eine -35-Consensusregion, eine -10-Consensus-region und eine Linkersequenz umfasst, die sich zwischen den Consensusregionen erstreckt,
worin die zumindest zwei verschiedenen Promotorkassetten Folgendes beinhalten: (i) eine erste Promotorkassette, die einen Promotor mit bekannter Sequenz umfasst, und eine zweite Promotorkassette, die einen durch Modifizierung der bekannten Sequenz entstandenen Promotor umfasst, oder (ii) eine erste und eine zweite Promotorkassette, die je einen durch Modifizierung derselben bekannten Sequenz entstandenen Promotor umfassen, und
eine 3'-Sequenz, die zu einer stromab flankierenden Region der Zielstelle homolog ist;
b) das Transformieren bakterieller Wirtszellen mit der Promotorenbibliothek, worin die Promotorkassetten in die bakteriellen Wirtszellen mittels homologer Rekombination zwischen den Promotorkassetten und den flankierenden Regionen der Zielstelle integriert werden, um transformierte Wirtszellen zu erzeugen, worin die Promotorkassette eine native Promotorregion eines chromosomalen Gens von Interesse ersetzt;
c) das Kultivieren der transformierten Wirtszellen unter geeigneten Kulturbedingungen; und
d) das Erhalten einer Bibliothek von transformierten Bakterienzellen, worin die transformierten Bakterienzellen einen Bereich von Ausmaßen an Expression des chromosomalen Gens von Interesse aufweisen.

2. Verfahren nach Anspruch 1, weiters umfassend das Selektieren transformierter Bakterienzellen aus der Bibliothek.

3. Verfahren nach Anspruch 1, worin die Wirtszellen aus der aus E. coli, Bacillus sp. und Pantoea sp. bestehenden Gruppe ausgewählt sind.

4. Verfahren nach Anspruch 2, worin:
(a) die selektierten Bakterienzellen ein höheres Ausmaß an Expression des Gens von Interesse aufweisen als Bakterienzellen, die den Wildtyp-Promotor umfassen; oder
(b) die selektierten Bakterienzellen ein geringeres Ausmaß an Expression des Gens von Interesse aufweisen als die Bakterienzellen, die den Wildtyp-Promotor umfassen.

5. Verfahren nach Anspruch 1, worin:
(a) die Promotorenbibliothek den Ptrc-Promotor und modifizierte Ptrc-Promotoren umfasst; oder
(b) die Promotorenbibliothek den Ptac-Promotor und modifizierte Ptrc-Promotoren umfasst; oder
(c) die Promotorenbibliothek den P_{GI}-Promotor und modifizierte P_{GI}-Promotoren umfasst; oder
(d) die Promotorenbibliothek modifizierte Promotoren mit Seq.-ID Nr. 28, Seq.-ID Nr. 29 und Seq.-ID Nr. 30 umfasst.

6. Vektor, umfassend eine Promotorkassette, wobei die Promotorkassette Folgendes in aufeinander folgender Reihenfolge umfasst:
(a) eine 5'-Sequenz, die zu einer stromauf flankierenden Region einer Zielstelle homolog ist;
(b) eine erste Rekombinaseerkennungsstelle;
(c) einen selektierbaren Marker;
(d) eine zweite Rekombinaseerkennungsstelle;
(e) einen modifizierten Promotor, der zumindest ein modifiziertes Nucleotid im Vergleich zu einem Wildtyp-Promotor an einer Position umfasst, die einer -35-Consensusregion, einer -10-Consensusregion oder einer Linkersequenz entspricht, die sich zwischen den Consensusregionen des Wildtyp-Protomors erstreckt, worin der Wildtyp-Promotor aus der aus P_{trc}, P_{tacl}, P_{D/E20}, P_{H207}, P_{N25}, P_{G25}, P_{J5}, P_{A1}, P_{A2}, P_{A3}, P_{L}, P_{lac}, P_{lacUVS}, P_{con} und P_{bla} bestehenden Gruppe ausgewählt ist; und
(f) eine 3'-Sequenz, die zu einer stromab flankierenden Region der Zielstelle homolog ist, worin homologe Rekombination zwischen der Promotorkassette und den flankierenden Regionen der Zielstelle dazu führt, dass das Promotorkonstrukt eine native Promotorregion eines chromosomalen Gens von Interesse ersetzt.

7. Vektor nach Anspruch 6, worin die Linkersequenz modifiziert ist.

8. Vektor nach Anspruch 6, worin die erste und die zweite Rekombinaseerkennungsstelle nicht identische Rekombinasestellen und aus lox und mutierten lox-Stellen ausgewählt sind.

9. Vektor nach Anspruch 6, worin der modifizierte Promotor aus der aus Seq.-ID Nr. 28 (NF-T), Seq.-ID Nr. 29 (NF-G), Seq.-ID Nr. 30 (NF-C), Seq.-ID Nr. 32 (NF-1T) und Seq.-ID Nr. 33 (NF-2T) bestehenden Gruppe ausgewählt ist.

10. Promotorenbibliothek, umfassend zumindest zwei verschiedene Promotorkassetten, worin die Promotorkassetten Folgendes in aufeinander folgender Reihenfolge umfassen:
eine 5'-Sequenz, die zu einer stromauf flankierenden Region einer Zielstelle homolog ist;
eine erste Rekombinaseerkennungsstelle;
einen selektierbaren Marker;
eine zweite Rekombinaseerkennungsstelle;
einen Promotor, der eine -35-Consensusregion, eine -10-Consensus-region und eine Linkersequenz umfasst, die sich zwischen den Consensusregionen erstreckt,
worin die zumindest zwei verschiedenen Promotorkassetten Folgendes beinhalten: (i) eine erste Promotorkassette, die einen Promotor mit bekannter Sequenz umfasst, und eine zweite Promotorkassette, die einen durch Modifizierung der bekannten Sequenz entstandenen Promotor umfasst, oder (ii) eine erste und eine zweite Promotorkassette, die je einen durch Modifizierung derselben bekannten Sequenz entstandenen Promotor umfassen, und
eine 3'-Sequenz, die zu einer stromab flankierenden Region der Zielstelle homolog ist.

11. Wirtszelle, transformiert mit einem Vektor nach einem der Ansprüche 6 bis 9.

12. Wirtszelle nach Anspruch 11, worin die Wirtszelle aus der aus E. coli, Bacillus sp. und Pantoea sp. bestehenden Gruppe ausgewählt ist.

13. Verfahren zur Modifizierung der regulierenden Funktion eines nativen Promotors eines chromosomalen Gens von Interesse, umfassend:
a) das Erhalten eines Vektors, der eine Promotorkassette nach einem der Ansprüche 6 bis 9 umfasst;
b) das Transformieren einer Wirtszelle mit dem Vektor, der eine Promotorkassette umfasst, um homologe Rekombination zwischen der Promotorkassette und homologen flankierenden Regionen einer Zielstelle zu ermöglichen, worin die Promotorkassette eine native Promotorregion eines chromosomalen Gens von Interesse ersetzt; und
c) das Kultivieren der transformierten Wirtszellen unter geeigneten Kulturbedingungen.

14. Verfahren nach Anspruch 13, weiters umfassend:
(a) das Ausschneiden des selektierbaren Markers aus der transformierten Wirtszelle und/oder
(b) das Isolieren der transformierten Wirtszellen.

15. Verfahren nach Anspruch 1, weiters umfassend:
(a) das Isolieren transformierter Wirtszellen mit einem gewünschten Ausmaß an Expression eines Gens von Interesse und/oder
(b) das Ausschneiden des selektierbaren Markers aus der transformierten Wirtszelle.

16. Verfahren zur Veränderung der Expression eines chromosomalen Gens von Interesse, umfassend:
a) das Erhalten eines Vektors, der eine Promotorkassette nach einem der Ansprüche 6 bis 9 umfasst;
b) das Transformieren einer Wirtszelle mit dem Vektor, der eine Promotorkassette umfasst; und
c) das Ermöglichen von homologer Rekombination zwischen der Promotorkassette und homologen flankierenden Regionen der Zielstelle, worin die Promotorkassette eine native Promotorregion eines chromosomalen Gens von Interesse ersetzt und die Expression des chromosomalen Gens von Interesse im Vergleich zu der Expression des chromosomalen Gens von Interesse in einer entsprechenden Stammwirtszelle verändert.

## Revendications

1. Méthode de création d'une bibliothèque de cellules bactériennes ayant une plage de niveaux d'expression d'un gène chromosomal d'intérêt comprenant,
a) obtenir une bibliothèque promoteur comprenant au moins deux différentes cassettes promoteur, où les cassettes promoteur comprennent dans l'ordre séquentiel:
une séquence 5' homologue à une région adjacente amont d'un site cible;
un premier site de reconnaissance de recombinase;
un marqueur pouvant être sélectionné;
un deuxième site de reconnaissance de recombinase;
un promoteur comprenant une région de consensus -35, une région de consensus -10 et une séquence de liaison s'étendant entre les régions de consensus, et où lesdites au moins deux cassettes promoteur différentes comprennent: (i) une première cassette promoteur comprenant un promoteur d'une séquence connue et une deuxième cassette promoteur comprenant un promoteur produit par la modification de ladite séquence connue; ou (ii) une première et une deuxième cassette promoteur comprenant chacune un promoteur produit par la modification de la même séquence connue; et
une séquence 3' homologue à une région adjacente aval du site cible,
b) transformer des cellules hôtes bactériennes avec la bibliothèque promoteur, où les cassettes promoteur sont intégrées dans les cellules hôtes bactériennes par une recombinaison homologue entre les cassettes de promoteur et les régions adjacentes du site cible pour produire des cellules hôtes transformées, où la cassette promoteur remplace une région promoteur native du gène chromosomal d'intérêt;
c) cultiver les cellules hôtes transformées sous des conditions de culture appropriées; et
d) obtenir une bibliothèque de cellules bactériennes transformées, où les cellules bactériennes transformées présentent une plage de niveaux d'expression du gène chromosomal d'intérêt.

2. Méthode selon la revendication 1, comprenant en outre la sélection de cellules bactériennes transformées de la librairie.

3. Méthode selon la revendication 1, dans laquelle les cellules hôtes sont sélectionnées dans le groupe consistant en *E. coli, Bacillus* sp. et *Pantoea* sp.

4. Méthode selon la revendication 2, dans laquelle:
(a) les cellules bactériennes sélectionnées ont un niveau d'expression plus élevé du gène d'intérêt que les cellules bactériennes comprenant le promoteur de type sauvage; ou
(b) les cellules bactériennes sélectionnées ont un niveau d'expression plus bas du gène d'intérêt que les cellules bactériennes comprenant le promoteur de type sauvage.

5. Méthode selon la revendication 1, dans laquelle:
(a) la bibliothèque promoteur comprend le promoteur Ptrc et les promoteurs Ptrc modifiés; ou
(b) la bibliothèque promoteur comprend le promoteur Ptrc et les promoteurs Ptrc modifiés; ou
(c) la bibliothèque promoteur comprend le promoteur P_{G1} et les promoteurs P_{G1} modifiés; ou
(d) la bibliothèque promoteur comprend des promoteurs modifiés ayant les SEQ ID NO. 28, SEQ ID NO. 29 et SEQ ID NO. 30.

6. Vecteur comprenant une cassette promoteur, la cassette promoteur comprenant dans l'ordre séquentiel (a) une séquence 5' homologue à une région adjacente amont d'un site cible;
(b) un premier site de reconnaissance de recombinase;
(c) un marqueur pouvant être sélectionné;
(d) un deuxième site de reconnaissance de recombinase;
(e) un promoteur modifié comprenant au moins un nucléotide modifié relativement à un promoteur de type sauvage dans une position correspondant à une région de consensus -35, une région de consensus -10 ou une séquence de liaison s'étendant entre les régions de consensus du promoteur de type sauvage, où le promoteur de type sauvage est sélectionné dans le groupe consistant en P_{trc}, P_{tacl}, P_{D/E20}, P_{H207}, P_{N25}, P_{G25}, P_{J5}, P_{A1}, P_{A2}, P_{A3}, P_{L}, P_{IAC}, P_{taUV5}, P_{con} et P_{bla}; et
(f) une séquence 3' homologue à une région adjacente aval du site cible, où la recombinaison homologue entre la cassette promoteur et les régions adjacentes du site cible se traduit par un produit de synthèse de promoteur remplaçant une région promoteur native d'un gène chromosomal d'intérêt.

7. Vecteur selon la revendication 6, où la séquence de liaison est modifiée.

8. Vecteur selon la revendication 6, où ledit premier et ledit deuxième site de reconnaissance de recombinase sont des sites de recombinase non identiques et sont sélectionnés parmi des sites d'oxygène liquide et d'oxygène liquide mutant.

9. Vecteur selon la revendication 6, où le promoteur modifié est sélectionné dans le groupe consistant en SEQ ID NO. 28 (NF-T), SEQ ID NO. 29 (NF-G), SEQ ID NO. 30 (NF-C), SEQ ID NO. 32 (NT-1T) et SEQ ID NO. 33 (NF-2T).

10. Bibliothèque promoteur comprenant au moins deux cassettes promoteur différentes, où les cassettes promoteur comprennent dans l'ordre séquentiel:
une séquence 5' homologue à une région adjacente amont d'un site cible;
un premier site de reconnaissance de recombinase;
un marqueur pouvant être sélectionné;
un deuxième site de reconnaissance de recombinase;
un promoteur comprenant une région de consensus -35, une région de consensus -10 et une séquence de liaison s'étendant entre les régions de consensus, et où lesdites au moins deux cassettes promoteur différentes comprennent: (i) une première cassette promoteur comprenant un promoteur d'une séquence connue et une deuxième cassette promoteur comprenant un promoteur produit par la modification de ladite séquence connue; ou (ii) une première et une deuxième cassette promoteur comprenant chacune un promoteur produit par la modification de la même séquence connue; et
une séquence 3' homologue à une région adjacente aval du site cible.

11. Cellule hôte transformée par un vecteur selon l'une quelconque des revendications 6 à 9.

12. Cellule hôte selon la revendication 11, où la cellule hôte est sélectionnée dans le groupe consistant en *E. coli, Bacillus sp* et *Pantoea sp.*

13. Méthode de modification d'une fonction régulatoire d'un promoteur natif d'un gène chromosomal d'intérêt comprenant:
a) obtenir un vecteur comprenant une cassette promoteur selon l'une quelconque des revendications 6 à 9;
b) transformer une cellule hôte avec le vecteur comprenant une cassette promoteur pour permettre une recombinaison homologue entre la cassette promoteur et des régions adjacentes homologues d'un site cible, où la cassette promoteur remplace une région promoteur native d'un gène chromosomal d'intérêt; et
c) cultiver les cellules hôtes transformées sous des conditions de culture appropriées.

14. Méthode selon la revendication 13, comprenant en outre:
(a) exciser le marqueur pouvant être sélectionné de la cellule hôte transformée; et/ou
(b) isoler les cellules hôtes transformées.

15. Méthode selon la revendication 1, comprenant en outre:
(a) isoler les cellules hôtes transformées ayant un niveau d'expression souhaité d'un gène d'intérêt et/ou
(b) exciser le marqueur pouvant être sélectionné de la cellule hôte transformée.

16. Méthode pour modifier l'expression d'un gène chromosomal d'intérêt comprenant:
a) obtenir un vecteur comprenant une cassette promoteur selon l'une quelconque des revendications 6 à 9;
b) transformer une cellule hôte avec le vecteur comprenant une cassette promoteur; et
c) permettre une recombinaison homologue entre la cassette promoteur et des régions avoisinantes homologues du site cible, où la cassette promoteur remplace une région promoteur native d'un gène chromosomal d'intérêt et modifie l'expression du gène chromosomal d'intérêt en comparaison avec l'expression du gène chromosomal d'intérêt dans une cellule hôte parent correspondante.
